# EUROPEAN PATENT APPLICATION

(11) **EP 4 079 764 A1**
(43) Date of publication of application: **26.10.2022**
(21) Application number: 20903156.6
(22) Date of filing: 21.12.2020
(51) Int. Cl.: C07K 19/00, C12N 15/62

(54) **LACTIC ACID OPTICAL PROBE, PREPARATION METHOD THEREFOR AND APPLICATION THEREOF**

(30) Priority: 19.12.2019 CN 201911318003
(71) Applicant: East China University of Science and Technology, Shanghai 200237 (CN)
(72) Inventor: YANG, Yi, Shanghai 200237 (CN); ZHAO, Yuzheng, Shanghai 200237 (CN); LI, Xie, Shanghai 200237 (CN); ZHANG, Xiuze, Shanghai 200237 (CN); HUANG, Li, Shanghai 200237 (CN)
(74) Representative: Coles, Andrea Birgit
(86) International application number: PCT/CN2020/137900
(87) International publication number: WO 2021/121417

(57) **Abstract**

An optical probe, comprising a lactic acid-sensitive polypeptide or a functional variant thereof and an optically active polypeptide or a functional variant thereof; the optically active polypeptide or the functional variant thereof is located in the sequence of the lactic acid-sensitive polypeptide or the functional variant thereof. A preparation method for the described probe and an application thereof in the detection of lactic acid.

## Description

### Technical field

The present invention relates to the field of optical probe technology, especially to a lactate optical probe, preparation method thereof and application thereof.

### Background

Lactate is a α-hydroxy acid, which could be ionized to form one hydrogen ion H+ in water, thereby forming CH₃CH(OH)COO- lactate ion. Lactate is a chiral molecule and consists of two enantiomers, D-(-)-lactate and L-(+)-lactate. In human body, lactate may exist in two different configurations, with L-lactate accounting for the majority. Lactic acidosis may occur when the concentration of L-lactate in plasma is greater than 5 mM and the blood pH is less than 7.35. Causes of lactic acidosis include deficiency of the intramitochondrial pyruvate transport network, pyruvate dehydrogenase complex defects, citric acid cycle disorders, congenital mitochondrial respiratory chain disorders, and tissue hypoxia. Lactate homeostasis is associated with glucose metabolism, and thus diabetes is associated with disorders of lactate metabolism. Compared with healthy controls, patients with diabetes have a decreased basal whole-body rate of glucose oxidation and an increased basal whole-body rate of non oxidative glycolysis, leading to the formation of excess lactate. The rising concentration of L-lactate in blood was once regarded as a waste product generated by skeletal muscle to provide energy via the glycolytic pathway under hypoxic conditions, and it is now widely accepted that a variety of different cells in body produce L-lactate under fully aerobic conditions which is used as fuel by virtually all tissues through the peripheral circulation. Lactate is the main energy source in the body, and the contribution of lactate in the process of tricarboxylic acid cycle exceeds that of glucose during lung tumor cell growth. Due to the large uptake of glucose by tumor cells, the glycolytic pathway is utilized under aerobic conditions to provide energy for the growth and proliferation of tumor cells while producing a large amount of the metabolite lactate. Lactate is a potent inhibitor of immune cell function and survival, leading to tumor cell escape from immune responses, illustrating the important interactions between tumor derived lactate and immune cells in the tumor environment.

The metabolic pathways involved in lactate metabolism are important for understanding the physiological response to exercise and the pathogenesis of common diseases such as diabetes and cancer. Either production or elimination of lactate relies on a reversible redox reaction catalyzed by lactate dehydrogenase (LDH). In animals during normal metabolism and exercise, glucose produces pyruvate via glycolysis and pyruvate continuously produces L-lactate via lactate dehydrogenase (LDH), but the concentration of lactate does not rise until the rate of lactate production exceeds its rate of elimination. During strenuous exercise, ATP is rapidly synthesized from glucose in blood by anaerobic respiration for body, while large amounts of pyruvate are produced and subsequently immediately converted to lactate, at which point the rate of lactate production is higher than the body's rate of lactate clearance, resulting in a rise in the concentration of lactate. Lactate in body has two paths, one is to regenerate pyruvate which enters the aerobic respiratory chain for oxidation supply; the other is to be converted into glucose in liver through gluconeogenesis, which enters the peripheral circulation. Lactate is the most predominant gluconeogenic precursor substance, and during intense exercise, glycogen within muscle cells forms pyruvate through glycolysis, and glucose-6-phosphate cannot be catalyzed to form glucose because there is no 6-phosphogluconase within muscle. Accordingly, after lactate diffuses through the cell membrane into the blood, it reenters the liver, becomes pyruvate under the action of hepatic lactate dehydrogenase, followed by glucose production via the gluconeogenic pathway, and returns to blood for the needs of muscle and the brain for glucose, this cycle is called the Cori-cycle, also called lactate cycle. Lactate upregulates hypoxia inducible factor 1α (HIF-1α) and vascular endothelial growth factor (VEGF), with indispensable roles in angiogenesis. Given the central role in lactate metabolism, lactate is now recognized as an integral part of primary and metastatic cancer metabolism, and several studies have shown that lactate plays a key role in tumor growth and metastatic disease progression.

Due to the above-mentioned important roles of lactate, the detection of lactate content is particularly important. Conventional detection methods for lactate include NaOH solution titration, high performance liquid chromatography (HPLC) (Bai Dong Mei, et al., Analytical Chemistry, 2001, 29 (4): 413-415), furthermore, optical rotator detection can also be employed because lactate is a chiral molecule and has optical rotatory characteristics; Ultraviolet (UV)-Enzymatic method (Immonen, K., et al., Meat Sci, 2000. 54 (2): p. 163-7.); Enzyme electrode assay (Tanner, R.K., et al., Eur J Appl Physiol, 2010. 109 (3): p.551-9). However, these methods are not suitable for live cell studies and have many drawbacks: they need time-consuming sample processing processes, such as cell disruption, isolation, and purification, etc; in situ, real-time, dynamic, high-throughput, and high spatiotemporal resolution assays cannot be performed in live cells and subcellular organelles. There remains a need in the field for methods that can detect lactate in situ, quantitatively, and at high throughput in real time inside and outside cells.

### Summary

The object of the invention is to provide probes and methods for in situ, high-throughput, quantitative detection of lactate in real time inside and outside cells.

To achieve the above object, the invention provides the following technical solutions:
The invention provides a lactate optical probe comprising a lactate-sensitive polypeptide or a functional variant thereof and an optically active polypeptide or a functional variant thereof, wherein the optically active polypeptide or the functional variant thereof is located in the sequence of the lactate-sensitive polypeptide or the functional variant thereof. The lactate-sensitive polypeptide or the functional variant thereof are divided into Part I and Part II by the optically active polypeptide or the functional variant thereof.

The invention provides a lactate optical probe comprising lactate-sensitive polypeptide B and optically active polypeptide A, wherein the optically active polypeptide A is located in the sequence of the lactate-sensitive polypeptide B, dividing the lactate-sensitive polypeptide B into the first part B1 and the second part B2, forming a probe structure of type B1-A-B2.

In one embodiment, the lactate-sensitive polypeptide comprises a lactate binding domain of a lactate binding protein. In one embodiment, the lactate-sensitive polypeptide is derived from *Escherichia coli.* In one embodiment, the lactate-sensitive polypeptide is a lactate binding protein or a functional fragment thereof. In one or more embodiments, the lactate binding protein is LldR protein. In one embodiment, the lactate-sensitive polypeptide has the sequence shown in SEQ ID No: 1 or has at least 35%, 40%, 50%, 60%, 70%, 80%, 85%, 90%, 95%, 99% sequence identity with it and retains lactate binding function. In one embodiment, the lactate-sensitive polypeptide has amino acids 80-258 of the sequence as shown in SEQ ID No: 1, or a sequence that has at least 35%, 40%, 50%, 60%, 70%, 80%, 85%, 90%, 95%, 99% sequence identity with it and retains lactate binding function.

In one embodiment, the optically active polypeptide is a fluorescent protein or a functional fragment or variant thereof. In one embodiment, the fluorescent protein is selected from the group consisting of yellow fluorescent protein (cpYFP as shown in SEQ ID No: 2), green fluorescent protein (cpGFP as shown in SEQ ID No: 3), blue fluorescent protein (cpBFP as shown in SEQ ID No: 4), and apple red fluorescent protein (cpmApple as shown in SEQ ID No: 5). Preferably, the optically active polypeptide is cpYFP. In one embodiment, the fluorescent protein has a sequence as shown in any of SEQ ID Nos: 2-5.

In one embodiment, the optical probe further comprises one or more linkers flanking the optically active polypeptide. The linkers described herein may be any amino acid sequence of any length. In one embodiment, the optically active polypeptide is flanked by a linker of no more than 5 amino acids, such as a linker of 0, 1, 2, 3, 4 amino acids. In one embodiment, the linker flanking the optically active polypeptide comprises amino acid Y. In one embodiment, linker Y is located N-terminal and/or C-terminal to the optically active polypeptide. In one embodiment, the optical probe is shown as follows: the first part B1 of the lactate-sensitive polypeptide -Y- optically active polypeptide A-the second part B2 of the lactate-sensitive polypeptide. In one embodiment, the present optical probe does not comprise a linker.

In one embodiment, the optically active polypeptide is located in the lactate-sensitive polypeptide at a position selected from the group consisting of residues 93-97, 119-121, 137-141, 158-161, 185-191, 208-210, and/or 230-232, the lactate-sensitive polypeptide is a functional fragment of a lactate binding protein shown as amino acids 80-258 of SEQ ID No: 1, wherein the numbering corresponds to the full length of the lactate binding protein. In one embodiment, the optically active polypeptide substitutes the lactate-sensitive polypeptide at one or more amino acids selected from the following positions: residues 93-97, 119-121, 137-141, 158-161, 185-191, 208-210, and/or 230-232, the lactate-sensitive polypeptide is a functional fragment of the lactate binding protein shown asamino acids 80-258 of SEQ ID No: 1, wherein the numbering corresponds to the full length of the lactate binding protein.

In one embodiment, the optically active polypeptides were located in the lactate-sensitive polypeptides at one or more sites selected from the group consisting of: 93/94, 93/95, 93/96, 93/97, 94/95, 94/96, 94/97, 95/96, 95/97, 96/97, 119/120, 119/121, 120/121, 137/138, 137/139, 137/140, 137/141, 138/139, 138/140, 138/141, 139/140, 139/141, 140/141, 158/159, 158/160, 158/161, 159/160, 159/161, 160/161, 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/191, 190/191, 208/209, 208/210, 209/210, 230/231, 230/232 and/or 231/232. Preferably, the optically active polypeptide is located in the lactate-sensitive polypeptide at one or more sites selected from the group consisting of: 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/191 or 190/191. In one or more embodiments, the type B1-A-B2 optical probe may be a probe wherein cpYFP is located at 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/191 or 190/191 of a lactate binding protein or a functional fragment thereof. In an exemplary embodiment, the type B1-A-B2 optical probe can be a probe wherein cpYFP is located at 185/186, 185/187, 185/188, 185/189, 185/190, 186/187, 186/188, 186/189, 186/190, 187/189, 189/191 and 190/191 of a lactate binding protein or a functional fragment thereof. In one or more embodiments, the functional fragment of the lactate binding protein is as shown in positions 80-258 of SEQ ID No: 1. In one embodiment, the present optical probe has or consists of the sequence shown in SEQ ID Nos: 6-17.

The invention also provides mutants of a lactate-sensitive polypeptide having one or more mutations. In one embodiment, the mutations are at positions 185, 189, and/or 190 of the lactate binding protein or a functional fragment thereof.

In one or more embodiments, the mutations comprise: P189R and P190D, P189R and P190A, P189R and P190I, P189R and P190Q, P189R and P190N, P189D and P190D, P189D and P190E, P189D and P190V, P189D and P190L, P189D and P190F, P189D and P190I, P189D and P190Q, P189D and P190N, P189D and P190G, P189D and P190Y, P189D and P190W, P189E and P190R, P189E and P190A, P189E and P190V, P189E and P190Q, P189A and P190L, P189A and P190F, P189A and P190M, P189A, P189A and P190N, P189A and P190G, P189A and P190H, P189A and P190T, P189V and P190D, P189V and P190E, P189V and P190A, P189V, P189V and P190N, P189V and P190H, P189V and P190Y, P189L and P190V, P189L and P190F, P189L and P190M, P189L and P190G, P189L and P190H, P189F and P190D, P189F and P190L, P189F and P190F, P189F and P190I, P189F and P190N, P189F and P190H, P189F and P190Y, P189F and P190K, P189F and P190T, P189F and P190W, P189I and P190R, P189I and P190D, P189I and P190A, P189I and P190V, P189I and P190M, P189I and P190Q, P189I and P190G, P189I and P190Y, P189I and P190S, P189I and P190T, P189M and P190R, P189M and P190D, P189M and P190E, P189M and P190F, P189M and P190G, P189M and P190S, P189M and P190W, P189C and P190D, P189C and P190E, P189C and P190F, P189C and P190I, P189C and P190M, P189C and P190C, P189C, P189C and P190H, P189C and P190Y, P189C and P190S, P189C and P190W, P190L, P190F, P190I, P190Q, P190N, P190K, P190T, P189Q and P190E, P189Q and P190A, P189Q and P190V, P189Q and P190M, P189Q and P190C, P189Q and P190Q, P189Q and P190H, P189Q and P190S, P189N and P190R, P189N and P190D, P189N and P190L, P189N and P190F, P189N and P190C, P189N, P189N and P190N, P189N and P190G, P189N and P190H, P189N and P190Y, P189N and P190T, P189G and P190V, P189G and P190F, P189G and P190M, P189G and P190C, P189G and P190G, P189G and P190H, P189G and P190K, P189G and P190W, P189H and P190R, P189H and P190D, P189H and P190E, P189H and P190L, P189H and P190S, P189Y and P190R, P189Y and P190L, P189Y and P190N, P189Y and P190H, P189Y and P190S, P189Y and P190T, P189K and P190D, P189K and P190E, P189K and P190V, P189K and P190L, P189K and P190F, P189K and P190I, P189K and P190M, P189K, P189K and P190Q, P189K and P190N, P189K and P190Y, P189K and P190K, P189K and P190T, P189S and P190E, P189S and P190A, P189S and P190L, P189S and P190F, P189S and P190M, P189S and P190C, P189S, P189S and P190Q, 189S and P190Y, P189S and P190K, P189S and P190S, P189T and P190R, P189T and P190D, P189T and P190M, P189T and P190C, P189T, P189T and P190Q, P189T and P190N, P189T and P190H, P189T and P190Y, P189T and P190K, P189T and P190W, P189W and P190A, P189W and P190V, P189W and P190F, P189W, P189W and P190Q, P189W and P190H, P189W and P190S, P189W and P190T, P189W and P190W. In one or more embodiments, the mutations further comprise: M185F, M185Y, M185L, M185I, M185Q, M185G, M185H, M185A, M185P, M185N, M185C, M185W, M185S, M185V, M185D, M185T, M185R, M185E or M185K; preferably, the mutations further comprise: M185F, M185Y, M185L, M185I, M185Q, M185G, M185H, M185A, M185N, M185C, M185W, M185S, M185V, M185D, M185T, M185R or M185K.

In one or more embodiments, the mutations comprise: P189R and P190A, P189D and P190D, P189D and P190E, P189D and P190Q, P189D and P190Y, P189A and P190N, P189A and P190G, P189V and P190H, P189F and P190I, P189F and P190N, P189F and P190K, P189I and P190D, P189I and P190A, P189I and P190V, P189I and P190M, P189M and P190R, P189M and P190E, P189M and P190F, P189M and P190G, P189M and P190S, P189C and P190E, P190Q, P189Q and P190M, P189Q and P190C, P189N and P190N, P189G and P190F, P189H and P190L, P189H and P190S, P189Y and P190L, P189K and P190V, P189K and P190T, P189S and P190A, P189S and P190M, P189S and P190Q, P189S and P190K, P189S and P190S, P189T and P190D, P189W and P190A, P189W and P190T, P189C and P190D, P189C and P190Y, P189N and P190Y, P189R and P190I, P189M and P190D, P189H and P190R, P189N, P189F and P190D, P189F and P190H, P189N and P190F, P189C and P190F, P189H and P190D, or P189S. In one or more embodiments, the mutations further comprise: M185F, M185Y, M185L, M185I, M185Q, M185G, M185H, M185A, M185P, M185N, M185C, M185W, M185S, M185V, M185D, M185T, M185R, M185E or M185K; preferably, the mutations further comprise: M185F, M185Y, M185L, M185I, M185Q, M185G, M185H, M185A, M185N, M185C, M185W, M185S, M185V, M185D, M185T, M185R or M185K.

In one ore more embodiments, the mutations comprise: P189S, P189C and P190D, P189C and P190Y, P189N and P190Y, P189R and P190I, P189M and P190D, P189H and P190R, P189N, P189F and P190D, P189F and P190H, P189N and P190F, P189C and P190F, or P189H and P190D. In one ore more embodiments, the mutations further comprise: M185F, M185Y, M185L, M185I, M185Q, M185G, M185H, M185A, M185P, M185N, M185C, M185W, M185S, M185V, M185D, M185T, M185R, M185E or M185K; preferably, the mutations further comprise: M185F, M185Y, M185L, M185I, M185Q, M185G, M185H, M185A, M185N, M185C, M185W, M185S, M185V, M185D, M185T, M185R or M185K.

In one ore more embodiments, the mutations comprise: (1) P189C and P190D, P189M and P190D, P189F and P190D, or P189H and P190D, and optionally (2) M185F, M185Y, M185L, M185I, M185Q, M185G, M185H, M185A, M185P, M185N, M185C, M185W, M185S, M185V, M185D, M185T, M185R, M185E or M185K.

In one ore more embodiments, the mutations comprise: (1) P189C and P190D, P189M and P190D, 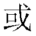 P189H and P190D, and optionally (2) M185F, M185Y, M185L, M185I, M185Q, M185G, M185H, M185A, M185P, M185N, M185C, M185W, M185S, M185V, M185D, M185T, M185R, M185E or M185K; alternatively, the mutations comprise: (1) P189F and P190D, and optionally (2) M185F, M185Y, M185L, M185I, M185Q, M185G, M185H, M185A, M185N, M185C, M185W, M185S, M185V, M185D, M185T, M185R or M185K.

The lactate-sensitive polypeptide in the present optical probe may be a lactate-sensitive polypeptide with one or more mutations described above. In one or more embodiments, an optical probe comprising a mutated lactate-sensitive polypeptide exhibits a response to lactate higher or lower than that of the unmutated counterpart.

In an exemplary embodiment, the present optical probe may be a probe having cpYFP inserted at 185/189 of the lactate binding protein and one or more mutations selected from the group consisting of: P189R and P190D, P189R and P190A, P189R and P190I, P189R and P190Q, P189R and P190N, P189D and P190D, P189D and P190E, P189D and P190V, P189D and P190L, P189D and P190F, P189D and P190I, P189D and P190Q, P189D and P190N, P189D and P190G, P189D and P190Y, P189D and P190W, P189E and P190R, P189E and P190A, P189E and P190V, P189E and P190Q, P189A and P190L, P189A and P190F, P189A and P190M, P189A, P189A and P190N, P189A and P190G, P189A and P190H, P189A and P190T, P189V and P190D, P189V and P190E, P189V and P190A, P189V, P189V and P190N, P189V and P190H, P189V and P190Y, P189L and P190V, P189L and P190F, P189L and P190M, P189L and P190G, P189L and P190H, P189F and P190D, P189F and P190L, P189F and P190F, P189F and P190I, P189F and P190N, P189F and P190H, P189F and P190Y, P189F and P190K, P189F and P190T, P189F and P190W, P189I and P190R, P189I and P190D, P189I and P190A, P189I and P190V, P189I and P190M, P189I and P190Q, P189I and P190G, P189I and P190Y, P189I and P190S, P189I and P190T, P189M and P190R, P189M and P190D, P189M and P190E, P189M and P190F, P189M and P190G, P189M and P190S, P189M and P190W, P189C and P190D, P189C and P190E, P189C and P190F, P189C and P190I, P189C and P190M, P189C and P190C, P189C, P189C and P190H, P189C and P190Y, P189C and P190S, P189C and P190W, P190L, P190F, P190I, P190Q, P190N, P190K, P190T, P189Q and P190E, P189Q and P190A, P189Q and P190V, P189Q and P190M, P189Q and P190C, P189Q and P190Q, P189Q and P190H, P189Q and P190S, P189N and P190R, P189N and P190D, P189N and P190L, P189N and P190F, P189N and P190C, P189N, P189N and P190N, P189N and P190G, P189N and P190H, P189N and P190Y, P189N and P190T, P189G and P190V, P189G and P190F, P189G and P190M, P189G and P190C, P189G and P190G, P189G and P190H, P189G and P190K, P189G and P190W, P189H and P190R, P189H and P190D, P189H and P190E, P189H and P190L, P189H and P190S, P189Y and P190R, P189Y and P190L, P189Y and P190N, P189Y and P190H, P189Y and P190S, P189Y and P190T, P189K and P190D, P189K and P190E, P189K and P190V, P189K and P190L, P189K and P190F, P189K and P190I, P189K and P190M, P189K, P189K and P190Q, P189K and P190N, P189K and P190Y, P189K and P190K, P189K and P190T, P189S and P190E, P189S and P190A, P189S and P190L, P189S and P190F, P189S and P190M, P189S and P190C, P189S, P189S and P190Q, 189S and P190Y, P189S and P190K, P189S and P190S, P189T and P190R, P189T and P190D, P189T and P190M, P189T and P190C, P189T, P189T and P190Q, P189T and P190N, P189T and P190H, P189T and P190Y, P189T and P190K, P189T and P190W, P189W and P190A, P189W and P190V, P189W and P190F, P189W, P189W and P190Q, P189W and P190H, P189W and P190S, P189W and P190T, P189W and P190W. In a further embodiment, the mutations further comprise: M185F, M185Y, M185L, M185I, M185Q, M185G, M185H, M185A, M185P, M185N, M185C, M185W, M185S, M185V, M185D, M185T, M185R, M185E or M185K; preferably, the mutations further comprise: M185F, M185Y, M185L, M185I, M185Q, M185G, M185H, M185A, M185N, M185C, M185W, M185S, M185V, M185D, M185T, M185R or M185K.

In an exemplary embodiment, the present optical probe may be a probe having cpYFP inserted at 185/189 of the lactate binding protein and having one or more mutations selected from the group consisting of: P189S, P189C and P190D, P189C and P190Y, P189N and P190Y, P189R and P190I, P189M and P190D, P189H and P190R, P189N, P189F and P190D, P189F and P190H, P189N and P190F, P189C and P190F, or P189H and P190D. In an exemplary embodiment, the functional fragment of the lactate binding protein is amino acid 80-259 of SEQ ID NO:1, wherein the mutations are P189N, P189S, P189C and P190F, P189N and P190F, P189N and P190Y, P189H and P190R, P189R and P190I, P189F and P190H, P189C and P190Y, P189C and P190D, P189M and P190D, P189H and P190D, or P189F and P190D. In a further embodiment, the mutations further comprise: M185F, M185Y, M185L, M185I, M185Q, M185G, M185H, M185A, M185P, M185N, M185C, M185W, M185S, M185V, M185D, M185T, M185R, M185E or M185K; preferably, the mutations further comprise: M185F, M185Y, M185L, M185I, M185Q, M185G, M185H, M185A, M185N, M185C, M185W, M185S, M185V, M185D, M185T, M185R or M185K.

In some particular embodiments, the present optical probe may be a probe having cpYFP inserted at 185/189 of the functional fragment of the lactate binding protein and having mutations, wherein the functional fragment of the lactate binding protein is amino acids 80-258 of SEQ ID No: 1, and the mutations comprise: (1) P189C and P190D, P189M and P190D, P189F and P190D, or P189H and P190D, and optionally (2) M185F, M185Y, M185L, M185I, M185Q, M185G, M185H, M185A, M185P, M185N, M185C, M185W, M185S, M185V, M185D, M185T, M185R, M185E or M185K. Preferably, the mutations comprise: (1) P189C and P190D, P189M and P190D, or P189H and P190D, and optionally (2) M185F, M185Y, M185L, M185I, M185Q, M185G, M185H, M185A, M185P, M185N, M185C, M185W, M185S, M185V, M185D, M185T, M185R, M185E or M185K; alternatively, the mutations comprise: (1) P189F and P190D, and optionally (2) M185F, M185Y, M185L, M185I, M185Q, M185G, M185H, M185A, M185N, M185C, M185W, M185S, M185V, M185D, M185T, M185R or M185K.

In one embodiment, the optical probes of the present invention have or consist of the sequence shown in SEQ ID Nos: 18-30 and 34-40.

The optical probes of the present invention comprise any of the amino acid sequence of SEQ ID Nos: 6-30 and 34-40 or a variant thereof. In one embodiment, the optical probes of the present invention comprise sequences having at least 35%, at least 40%, at least 50%, at least 60%, at least 70%, at least 80%, at least 85%, at least 90%, at least 95%, at least 99% sequence identity with any one of the amino acid sequences of SEQ ID Nos: 6-30 and 34-40. In preferred embodiments, the optical probes of the present invention consist of any sequence shown in SEQ ID Nos: 6-30 and 34-40. In more preferred embodiments, the optical probes of the present invention comprise or consist of SEQ ID Nos: 30 and 34-40.

The invention also provides fusion polypeptides comprising the optical probes described herein and other polypeptides. In some embodiments, other polypeptides are located N- and/or C-terminal to the said optical probe. In some embodiments, other polypeptides include polypeptides that localize optical probes to different organelles or subcellular organelles, tags for purification, or tags for immunoblotting.

The invention also provides nucleic acid sequences comprising the coding sequences for the polypeptide, probe, or protein described herein or a complementary sequence or fragment thereof. In one embodiment, the nucleic acid sequence of the present invention is selected from the group consisting of (1) the coding sequence of any of the amino acid sequences shown in SEQ ID Nos: 6-30 and 34-40 or complementary sequences thereof, (2) the sequences with at least 99%, 95%, 90%, 80%, 70% or 50% identity with (1), (3) the fragments of (1) or (2). In one embodiment, the nucleic acid sequence of the invention comprises SEQ ID No: 34 or a variant or fragment thereof. In one or more embodiments, the fragments are primers.

The invention also relates to complementary sequences of the nucleic acid sequences described above or variants thereof, including nucleic acid sequences encoding the fragments, analogs, derivatives, soluble fragments and variants of the optical probes or fusion proteins of the invention or the complementary sequences thereof.

The invention also provides nucleic acid constructs comprising the nucleic acid sequences described herein, or their complementary sequences, which encode the optical probes or fusion polypeptides described herein. In one or more embodiments, the nucleic acid construct is a cloning vector, expression vector, or recombinant vector. In one or more embodiments, the nucleic acid sequence is operably linked to an expression control sequence. In some embodiments, the expression vector is selected from the group consisting of prokaryotic expression vector, eukaryotic expression vector, and viral vector.

The invention also provides cells comprising the nucleic acid sequences or nucleic acid constructs described in the invention. In one or more embodiments, the cells express an optical probe or fusion polypeptide described herein.

The invention also provides a detection test kit comprising an optical probe or fusion polypeptide or polynucleotide described herein, or an optical probe or fusion polypeptide prepared according to the method described herein.

The invention provides methods for preparing the optical probes described herein, comprising providing cells expressing the optical probes or fusion polypeptides described herein, culturing the cells under conditions in which the cells express them, and isolating the optical probes or fusion polypeptides.

The present invention also provides a method for preparing the lactate optical probe described above, comprising the steps of 1) transferring an expression vector encoding the lactate optical probe described herein into a host cell; 2) culturing the host cells under conditions suitable for expression of the expression vector; 3) isolating the lactate optical probe.

The invention also provides a method for detecting lactate in a sample, comprising contacting the sample with an optical probe or fusion polypeptide described herein, or an optical probe or fusion polypeptide prepared according to the method described herein, and detecting changes in an optically active polypeptide. The detecting may be performed in vivo, in vitro, subcellularly, or in situ. Said sample is such as blood.

The invention also provides a method for quantitating lactate in a sample, comprising contacting the sample with an optical probe or fusion polypeptide described herein or an optical probe or fusion polypeptide prepared according to the method described herein, detecting changes in optically active polypeptide, and quantitating lactate in the sample according to the changes in optically active polypeptide.

The invention also provides a method for screening compounds, such as pharmaceuticals, comprising contacting a candidate compound with an optical probe or fusion polypeptide described herein, or an optical probe or fusion polypeptide prepared as described herein, detecting changes in the optically active polypeptide, and screening the compounds according to the changes in the optically active polypeptide. Said method allows high-throughput screening of compounds.

The invention also provides use of a lactate optical probe or fusion polypeptide described herein or a lactate optical probe or fusion polypeptide prepared according to the methods described herein in intracellular/extracellular localization of lactate. In one or more embodiments, the localization is in real time.

Beneficial effects of the present invention: the lactate optical probe of the present invention is easy to mature, has large dynamic change in fluorescence and good specificity, and is able of being expressed in cells by a genetically manipulated method, allows real time in situ, high-throughput, and quantitative detection of lactate inside and outside cells, eliminates the time-consuming sample processing steps. Experimental results show that the highest response to lactate by the lactate optical probe of the present application is more than 13 fold higher than that of the control, and that the localized, qualitative, and quantitative detection can be performed in subcellular structures such as cytosol, mitochondria, nucleus, endoplasmic reticulum, lysosomes, and Golgi apparatus, and high throughput screening of compounds as well as quantitative detection of lactate in blood may be performed.

### Description of drawings

The invention is further illustrated below in combination with the drawings and examples.
Fig. 1 is the SDS-PAGE of the exemplary lactate optical probe described in Example 1;
Fig. 2 is a lactate response change plot for an exemplary lactate optical probe comprising cpYFP and a lactate binding protein as described in Example 2;
Fig. 3 is a lactate response change plot for an exemplary lactate optical probe comprising cpGFP and a lactate binding protein as described in Example 3;
Fig. 4 is a lactate response change plot for an exemplary lactate optical probe comprising cpBFP and a lactate binding protein as described in Example 4;
Fig. 5 is a lactate response change plot for an exemplary lactate optical probe comprising cpmApple and a lactate binding protein as described in Example 5;

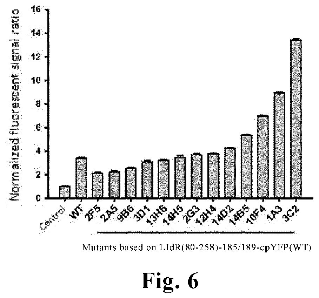

Fig. 6 is the response to lactate for a mutant of a lactate optical probe having cpYFP inserted at 185/189 of a lactate binding protein as described in Example 6;
Fig. 7A is a titration curve of different concentrations of lactate for an exemplary mutant of a lactate optical probe having cpYFP inserted at 185/189 of a lactate binding protein as described in Example 7; Fig. 7B is a titration curve of different concentrations of lactate for a mutant of a lactate optical probe described in Example 8;
Fig. 8 is a plot of the fluorescence spectral properties of an exemplary lactate optical probe described in Example 9;
Fig. 9 is the histogram of the specific detection of the exemplary lactate optical probe described in Example 9;
Fig. 10 is a picture of the subcellular organelle localization in mammalian cells of the exemplary lactate optical probe described in Example 10;
Fig. 11 is a schematic of the dynamic monitoring of lactate transmembrane transport in mammalian cells for the exemplary lactate optical probe described in Example 11;
Fig. 12 is a dot plot for high-throughput compound screening at the live cell level with an exemplary lactate optical probe described in Example 12;
Fig. 13 is a bar graph of the quantification of lactate in mouse and human blood with the exemplary lactate optical probe described in Example 13.

### Embodiments

When a value or range is given, the term "about" used herein means that the value or range is within 20%, within 10%, and within 5% of the given value or range.

The terms "comprise", "include", and equivalent forms thereof include the meaning of "contain" as well as "consist of', for example a composition "comprising" X may consist of X alone or may contain other substances, such as X + Y.

The term "lactate-sensitive polypeptide" or "lactate-responsive polypeptide" used herein refers to a polypeptide which responds to lactate production including any response in chemical, biological, electrical, or physiological parameters of a polypeptide that interacts with the sensitive-polypeptide. The response includes small changes, for example, changes in the orientation of the amino acid or peptide fragment of the polypeptide and, for example, changes in the primary, secondary or tertiary structure of the polypeptide, including, for example, changes in protonation, electrochemical potential and/or conformation. "Conformation" is the three-dimensional arrangement of the primary, secondary, and tertiary structure of a molecule containing side groups in the molecule; when the three-dimensional structure of the molecule changes, the conformation changes. Examples of conformational changes include transitions from α-helix to β-fold or from β-fold to α-helix. It should be understood that detectable alterations need not be conformational changes as long as the fluorescence of the fluorescent protein moiety is altered. The lactate-sensitive polypeptide described herein may also include functional variants thereof. The functional variants of the lactate-sensitive polypeptide include, but are not limited to, variants that can interact with lactate and thereby undergo the same or similar changes as the parent lactate-sensitive polypeptide.

The lactate-sensitive polypeptide of the present invention includes, but is not limited to, lactate binding protein LldR or its variants having more than 90% homology to it. The exemplary lactate binding protein described herein, LldR, is derived from *E*. *coli* JM109. LldR is a bacterial transcription factor consisting of a lactate binding/regulatory domain and a DNA binding domain. An exemplary LldR protein is shown in SEQ ID No: 1. In one or more embodiments, the lactate-sensitive polypeptide comprises a functional fragment (a lactate binding domain), i.e., amino acids 80-258 of a lactate binding protein. When describing the optical probes or lactate binding proteins of the invention (e.g., when describing insertion sites or mutation sites), the amino acid residues are all numbered according to SEQ ID No: 1.

The term "optical probe" used herein refers to a lactate-sensitive polypeptide fused to an optically active polypeptide. The inventors discovered that the conformational changes produced by binding a lactate-sensitive polypeptide, such as a lactate binding protein to a physiological concentration of lactate specifically by lactate sensitive polypeptides causes conformational changes in an optically active polypeptides, such as fluorescent proteins, which in turn result in the alteration of the optical properties of the optically active polypeptides. A standard curve is plotted with the help of the fluorescence of fluorescent proteins determined at different concentrations of lactate allows the presence and/or level of lactate to be detected and analyzed.

In the present optical probe, an optically active polypeptide, such as a fluorescent protein, is operably inserted into a lactate-sensitive polypeptide. The protein-based "optically active polypeptides" are polypeptides with the ability to emit fluorescence. Fluorescence is one optical property of an optically active polypeptide that can be used as a means of detecting the responsiveness of the optical probes of the present invention. Preferably, the protein substrate is selected to have fluorescence properties that are easily distinguishable in the unactivated and activated conformational states. The optically active polypeptide described herein may also include a functional variant thereof. A functional variant of the optically active polypeptide includes, but is not limited to, a variant for which the same or similar change in fluorescence property may occur as the parent optically active polypeptide.

The term "fluorescent protein" used herein refers to a protein that fluoresces upon irradiation with excitation light. Fluorescent proteins have been used as basic detection means in the field of biological sciences, such as green fluorescent protein (GFP) commonly used in biotechnology and circularly rearranged blue fluorescent protein (cpBFP), circularly rearranged green fluorescent protein (cpGFP), circularly rearranged yellow fluorescent protein (cpYFP) derived from mutations in this protein, etc; there are also a red fluorescent protein (RFP) commonly used in the art, and circularly rearranged proteins derived from this protein, such as cpmApple, cpmOrange, cpmKate, and others. Those skilled in the art know fluorescent proteins and sequences thereof that can be used for the invention. Exemplarily, cpYFP as shown in SEQ ID No: 2; cpGFP as shown in SEQ ID No: 3; cpBFP as shown in SEQ ID No: 4; and cpmApple as shown in SEQ ID No: 5.

A "linker" or "linker region" refers to an amino acid or nucleotide sequence linking two parts in a polypeptide, protein, or nucleic acid of the invention. Exemplarily, the number of amino acids at the amino terminus of the linker region of the lactate-sensitive polypeptide to the optically active polypeptide in the invention is selected to be 0-3, and the number of amino acids at the carboxyl terminus is selected to be 0-2; when a recombinant optical probe is linked to a functional protein as a basic unit, it can be fused at the amino or carboxyl terminus of the recombinant optical probe. The linker sequence may be a short peptide chain composed of one or more flexible amino acids, as in Y.

The lactate optical probes of the invention include lactate-sensitive polypeptide B, such as a lactate binding protein or its lactate binding domain (positions 80-258) or variants, and optically active polypeptide A, such as a fluorescent protein. The optically active polypeptide A is inserted into the lactate sensitive polypeptide B, and B was divided into two parts, B1 and B2, to form the probe structure of the formula B1-A-B2; the interaction between the lactate-sensitive polypeptide B and lactate results in a stronger optical signal for optically active polypeptide A.

In the present optical probe, the optically active polypeptide may be located anywhere in the lactate-sensitive polypeptide. In one embodiment, the optically active polypeptide is located anywhere in N-C direction over a lactate-sensitive polypeptide in N-C direction. Specifically, the optically active polypeptide is located in a flexible region of a lactate-sensitive polypeptide that refers to some specific structures present in higher structures of a protein such as loop domains, which are more mobile and flexible than other higher structures of the protein, and this region may undergo dynamic changes in spatial structural conformation upon binding of this protein and a ligand. The flexible regions described herein mainly refer to the regions where insertion sites in lactate binding proteins are located, such as regions of amino acid residues 93-97, 119-121, 137-141, 158-161, 185-191, 208-210 and/or 230-232. Exemplarily, the optically active polypeptides are located in the lactate binding protein or a functional fragment thereof at one or more sites selected from the group consisting of: 93/94, 93/95, 93/96, 93/97, 94/95, 94/96, 94/97, 95/96, 95/97, 96/97, 119/120, 119/121, 120/121, 137/138, 137/139, 137/140, 137/141, 138/139, 138/140, 138/141, 139/140, 139/141, 140/141, 158/159, 158/160, 158/161, 159/160, 159/161, 160/161, 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/191, 190/191, 208/209, 208/210, 209/210, 230/231, 230/232 or 231/232. In the present invention, if the two numbers in a site represented in "X/Y" form are consecutive integers, this means that the optically active polypeptide is located between the amino acids stated by those numbers. For example, insertion site 93/94 indicates that the optically active polypeptide is located between amino acids 93 and 94 of the lactate-sensitive polypeptide. If the two numbers in a site represented in "X/Y" form are not consecutive integers, it indicates that the optically active polypeptide displaces the amino acids between the amino acids indicated by those numbers. For example, insertion site 93/97 indicates the replacement of amino acids 94-96 of the lactate-sensitive polypeptide by an optically active polypeptide. In one embodiment, the optically active polypeptide is located in a lactate binding protein or a functional fragment thereof at one or more sites selected from the group consisting of: 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/191 or 190/191. In one embodiment, the optically active polypeptide is located in a lactate binding protein or a functional fragment thereof at one or more sites selected from the group consisting of: 185/186, 185/187, 185/188, 185/189, 185/190, 186/187, 186/188, 186/189, 186/190, 187/189, 189/191 and 190/191. In one embodiment, the optically active polypeptide is located in a lactate binding protein or a functional fragment thereof at one or more sites selected from the group consisting of: 185/189, 186/189 or 187/189. Preferably, the present optical probe may be a probe having cpYFP located at 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/191 or 190/191 of a lactate binding protein or a functional fragment thereof. In an exemplary embodiment, the present optical probe may be a probe having cpYFP located at 185/186, 185/187, 185/188, 185/189, 185/190, 186/187, 186/188, 186/189, 186/190, 187/189, 189/191 and 190/191 of a lactate binding protein or a functional fragment thereof. In one or more embodiments, the functional fragment of the lactate binding protein is as shown in positions 80-258 of SEQ ID No: 1. In one embodiment, the present optical probe has or consists of the sequence shown in SEQ ID Nos: 6-17.

When referring to a certain polypeptide or protein, the term "variant" or "mutant" used herein includes variants that have the same function of said polypeptide or protein but differ in sequence. These variants include, but are not limited to: deletions, insertions and/or substitutions of one or more (usually 1-30, preferably 1-20, more preferably 1-10, most preferably 1-5) amino acids in the sequence of the polypeptide or protein, and sequences obtained by adding one or more (usually within 20, preferably within 10, more preferably within 5) amino acids to its carboxy terminus and/or amino terminus. Not limited by theory, an amino acid residue is altered without changing the overall configuration and function of the polypeptide or protein, i.e., a functionally conserved mutation. For example, in the art, substitution with amino acids of approaching or similar perperty generally does not alter the function of a polypeptide or protein. In the art, amino acids with similar property tend to refer to families of amino acids with similar side chains, which have been well defined in the art. These families include amino acids with basic side chain (e.g., lysine, arginine, histidine), amino acids with acidic side chain (e.g., aspartic acid, glutamic acid), amino acids with uncharged polar side chain (e.g., glycine, asparagine, glutamine, serine, threonine, tyrosine, cysteine), amino acids with non-polar side chain (e.g., alanine, valine, leucine, isoleucine, lactate, phenylalanine, methionine, tryptophan), amino acids with β-branched side chain (e.g., threonine, valine, isoleucine) and amino acids with aromatic side chain (e.g., tyrosine, phenylalanine, tryptophan, histidine). For another example, the addition of one or more amino acids to the amino terminus and/or carboxy terminus also generally does not alter the function of a polypeptide or protein. The conservative amino acid substitutions of many commonly known non-genetic coding amino acids are known in the art. Conservative substitutions of other non-coding amino acids can be determined based on a comparison of their physical properties with those of the amino acids that are genetically encoded.

In two or more sequences of a polypeptide or nucleic acid molecule, the terms "identity" or "percent identity" refer to when the maximum correspondence is compared and aligned by manual alignment and visual inspection using methods known in the art such as sequence comparison algorithms, in a comparison window or a specified region, two or more sequences or subsequences are identical or have a certain percentage of amino acid residues or nucleotides that are identical in the specified region (e.g., 60%, 65%, 70%, 75%, 80%, 85%, 90%, 91%, 92%, 93%, 94%, 95%, 96%, 97%, 98%, 99%, or 100% identical). For example, the preferred algorithms suitable for determination of percent identity and percent similarity are the BLAST and BLAST 2.0 algorithms, respectively, see Altschul et al., (1977) nucleic acids res. 25:3389 and Altschul et al., (1990) J. mol. Biol 215:403.

Those skilled in the art is well known that in gene cloning operation, it is often necessary to design a suitable restriction enzyme cutting site, which is bound to introduce one or more incoherent residues at the ends of the expressed polypeptide or protein, and this does not affect the activity of the polypeptide or protein of interest. Also for constructing fusion proteins, facilitating the expression of recombinant proteins, obtaining recombinant proteins that are automatically secreted outside the host cell, or facilitating the purification of recombinant proteins, it is often necessary to add some amino acids to the N-terminus, C-terminus, or other suitable regions within the recombinant protein, for example, including but not limited to, suitable linker peptides, signal peptides, leader peptides, terminal extensions, glutathione S-transferases (GSTs) maltose E-binding protein, Protein A, tags such as 6His or flag, or the proteolytic enzyme sites of Factor Xa or thrombin or enterokinase.

The present optical probe may comprise a lactate-sensitive polypeptide with a mutation. The mutation may be located at P189 and/or P190 sites and, optionally, M185 site of the lactate binding protein or a functional fragment thereof.

Exemplarily, in one or more embodiments, the mutations are selected from the group consisting of: P189R and P190D, P189R and P190A, P189R and P190I, P189R and P190Q, P189R and P190N, P189D and P190D, P189D and P190E, P189D and P190V, P189D and P190L, P189D and P190F, P189D and P190I, P189D and P190Q, P189D and P190N, P189D and P190G, P189D and P190Y, P189D and P190W, P189E and P190R, P189E and P190A, P189E and P190V, P189E and P190Q, P189A and P190L, P189A and P190F, P189A and P190M, P189A, P189A and P190N, P189A and P190G, P189A and P190H, P189A and P190T, P189V and P190D, P189V and P190E, P189V and P190A, P189V, P189V and P190N, P189V and P190H, P189V and P190Y, P189L and P190V, P189L and P190F, P189L and P190M, P189L and P190G, P189L and P190H, P189F and P190D, P189F and P190L, P189F and P190F, P189F and P190I, P189F and P190N, P189F and P190H, P189F and P190Y, P189F and P190K, P189F and P190T, P189F and P190W, P189I and P190R, P189I and P190D, P189I and P190A, P189I and P190V, P189I and P190M, P189I and P190Q, P189I and P190G, P189I and P190Y, P189I and P190S, P189I and P190T, P189M and P190R, P189M and P190D, P189M and P190E, P189M and P190F, P189M and P190G, P189M and P190S, P189M and P190W, P189C and P190D, P189C and P190E, P189C and P190F, P189C and P190I, P189C and P190M, P189C and P190C, P189C, P189C and P190H, P189C and P190Y, P189C and P190S, P189C and P190W, P190L, P190F, P190I, P190Q, P190N, P190K, P190T, P189Q and P190E, P189Q and P190A, P189Q and P190V, P189Q and P190M, P189Q and P190C, P189Q and P190Q, P189Q and P190H, P189Q and P190S, P189N and P190R, P189N and P190D, P189N and P190L, P189N and P190F, P189N and P190C, P189N, P189N and P190N, P189N and P190G, P189N and P190H, P189N and P190Y, P189N and P190T, P189G and P190V, P189G and P190F, P189G and P190M, P189G and P190C, P189G and P190G, P189G and P190H, P189G and P190K, P189G and P190W, P189H and P190R, P189H and P190D, P189H and P190E, P189H and P190L, P189H and P190S, P189Y and P190R, P189Y and P190L, P189Y and P190N, P189Y and P190H, P189Y and P190S, P189Y and P190T, P189K and P190D, P189K and P190E, P189K and P190V, P189K and P190L, P189K and P190F, P189K and P190I, P189K and P190M, P189K, P189K and P190Q, P189K and P190N, P189K and P190Y, P189K and P190K, P189K and P190T, P189S and P190E, P189S and P190A, P189S and P190L, P189S and P190F, P189S and P190M, P189S and P190C, P189S, P189S and P190Q, 189S and P190Y, P189S and P190K, P189S and P190S, P189T and P190R, P189T and P190D, P189T and P190M, P189T and P190C, P189T, P189T and P190Q, P189T and P190N, P189T and P190H, P189T and P190Y, P189T and P190K, P189T and P190W, P189W and P190A, P189W and P190V, P189W and P190F, P189W, P189W and P190Q, P189W and P190H, P189W and P190S, P189W and P190T, P189W and P190W. In some specific embodiments, the mutations are selected from the group consisting of: P189R and P190A, P189D and P190D, P189D and P190E, P189D and P190Q, P189D and P190Y, P189A and P190N, P189A and P190G, P189V and P190H, P189F and P190I, P189F and P190N, P189F and P190K, P189I and P190D, P189I and P190A, P189I and P190V, P189I and P190M, P189M and P190R, P189M and P190E, P189M and P190F, P189M and P190G, P189M and P190S, P189C and P190E, P190Q, P189Q and P190M, P189Q and P190C, P189N and P190N, P189G and P190F, P189H and P190L, P189H and P190S, P189Y and P190L, P189K and P190V, P189K and P190T, P189S and P190A, P189S and P190M, P189S and P190Q, P189S and P190K, P189S and P190S, P189T and P190D, P189W and P190A, P189W and P190T, 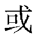 P189S, P189C and P190D, P189C and P190Y, P189N and P190Y, P189R and P190I, P189M and P190D, P189H and P190R, P189N, P189F and P190D, P189F and P190H, P189N and P190F, P189C and P190F, or P189H and P190D. The optical probe comprising above mutations responds more than 1.5-fold to lactate as that of control. In some embodiments, the mutations are one or more selected from the group consisting of: P189S, P189C and P190D, P189C and P190Y, P189N and P190Y, P189R and P190I, P189M and P190D, P189H and P190R, P189N, P189F and P190D, P189F and P190H, P189N and P190F, P189C and P190F, or P189H and P190D. The optical probe comprising above mutations responds more than 2-fold to lactate as that of control. In a preferred embodiment, the mutations are P189C and P190D, P189M and P190D, P189H and P190D or P189F and P190D.

In a further embodiment, the mutations further comprise M185F, M185Y, M185L, M185I, M185Q, M185G, M185H, M185A, M185P, M185N, M185C, M185W, M185S, M185V, M185D, M185T, M185R, M185E or M185K. In a further preferred embodiment, the mutations further comprise M185F, M185Y, M185L, M185I, M185Q, M185G, M185H, M185A, M185N, M185C, M185W, M185S, M185V, M185D, M185T, M185R or M185K.

The terms "functional variant", "derivative", and "analog" used herein refer to proteins that maintain essentially the same biological function or activity as the original polypeptide or protein (e.g., a lactate binding protein or a fluorescent protein). Functional variants, derivatives, or analogues of a polypeptide or protein (e.g., a lactate binding protein or a fluorescent protein) of the invention may be (i) a protein having one or more conservative or nonconservative amino acid residues (preferably conservative amino acid residues) substituted, whereas such substituted amino acid residues may or may not be encoded by the genetic code, or (ii) a protein having a substituted group in one or more amino acid residues, or (iii) a protein formed by the fusion of the mature protein to another compound, such as a compound that extends the half-life of the protein, such as polyethylene glycol, or (iv) a protein formed by the fusion of an additional amino acid sequence to this protein sequence (such as a secretory sequence or the sequence or protein used to purify this protein, or the fusion protein formed with an antigenic IgG fragment). According to the teaching herein, these functional variants, derivatives, and analogues belong to the common knowledge to those skilled in the art.

The difference of said analogues from the original polypeptide or protein may be a difference in amino acid sequence, a difference in modified form that does not affect the sequence, or both. These proteins include natural or induced genetic variants. Induced variants can be derived by various techniques, such as random mutagenesis by radiation or exposure to mutagens and can also be obtained by site directed mutagenesis or other known techniques in molecular biology.

The analogues also include analogues with a residue (such as a D-amino acid) different from the natural L-amino acid, as well as those with a non-natural or synthetic amino acids (such as β, γ-amino acids). It is understood that the lactate-sensitive polypeptides of the present invention are not limited to the representative proteins, variants, derivatives, and analogues above. Modified (usually without changing the primary structure) forms include chemically derived forms of the protein such as acetylated or carboxylated forms, in vivo or in vitro. Modifications also include glycosylation, such as those resulting from glycosylation modifications either in the synthesis and processing of the protein or in further processing steps. This modification can be accomplished by exposing the protein to enzymes for glycosylation, such as mammalian glycosylases or deglycosylases. The modified forms also include sequences with phosphorylated amino acid residues such as phosphotyrosine, phosphoserine, phosphothreonine. Proteins that were modified to improve their antiproteolytic properties or optimize the solubilization properties were also included.

The present fusion polypeptide comprises the optical probe described herein and an additional polypeptide. In some embodiments, the optical probe described herein also comprises another polypeptide fused to it. The additional polypeptide described herein does not affect the property of the optical probe. The additional polypeptide may be located N- and/or C-terminal to the optical probe. In some embodiments, the additional polypeptide includes polypeptides that localize the optical probe to different organelles or subcellular organelles, a tag for purification, or a tag for immunoblotting. There may be a linker between the optical probe and the additional polypeptide in the fusion polypeptide described herein.

The subcellular organelles described here include cytosol, mitochondria, nucleus, endoplasmic reticulum, cell membrane, Golgi apparatus, lysosome, and peroxisome, among others. In some embodiments, the tag for purification or the tag for immunoblotting include 6 histidine (6^{∗}His), glutathione sulfurtransferase (GST), Flag.

The terms "nucleic acid" or "nucleotide" or "polynucleotide" or "nucleic acid sequence" used herein may be in the form of DNA or in the form of RNA. DNA form includes cDNA, genomic DNA, or artificially synthesized DNA. DNA can be single stranded or double stranded. DNA can be either the coding or noncoding strand. When referring to nucleic acids, the term "variant" used herein may be a naturally occurring allelic variant or a non-naturally occurring variant. These nucleotide variants include degenerate variants, substitution variants, deletion variants, and insertion variants. As is known in the art, an allelic variant is a form of substitution of a nucleic acid, which may be a substitution, deletion, or insertion of one or more nucleotides but does not substantially alter the function of the protein it encodes. The nucleic acid of the invention may comprise a nucleotide sequence having sequence identity of at least about 70%, at least about 75%, at least about 80%, at least about 85%, at least about 90%, at least about 95%, at least about 98%, at least about 99%, or 100% to the nucleic acid sequence. The invention also relates to a nucleic acid fragment that hybridizes to the sequences described above. In an exemplary embodiment, the nucleic acid sequence is shown in SEQ ID No: 31, which represents the coding sequence of a probe having cpYFP inserted at 185/189 sites of a functional fragment of a lactate binding protein and having P189F/P190D mutations. As used herein, a "nucleic acid fragment" is at least 15 nucleotides in length, preferably at least 30 nucleotides, more preferably at least 50 nucleotides, and even more preferably at least 100 nucleotides or more. The nucleic acid fragment can be used in amplification techniques of nucleic acids (such as PCR).

The full-length sequences or fragments thereof of the optical probes or fusion proteins of the invention can often be obtained by PCR amplification, artificial synthesis or recombinant procedures. For PCR amplification, primers can be designed according to the nucleotide sequences disclosed in the invention and the sequences in interest can be amplified with a commercially available cDNA library or a cDNA library prepared by conventional methods known to those skilled in the art as template. When the nucleotide sequence is greater than 2500 bp, it is preferable to perform 2 ~ 6 rounds of PCR amplification, and then the individually amplified fragments are spliced together in the correct order. There is no special limit to the procedures and systems for PCR amplification described herein, and conventional PCR amplification procedures and systems in the art may be used. Recombination can also be used to obtain relevant sequences in bulk. This is usually by cloning into a vector, retransferring into cells, and then isolating and purifying the polypeptide or protein in inserest from proliferated host cells by conventional methods. In addition, synthetic methods are also available to synthesize the the sequence in interest, especially for short fragments. In the present invention, when the nucleotide sequence of the optical probe is less than 2500 bp, it may be synthesized by an artificial synthetic method. The artificial synthetic method is a conventional artificial synthetic method for DNA in the art with no other special requirements. In general, many small fragments are first synthesized, and then ligated to obtain a very long sequence. At present, it has become possible to obtain, entirely by chemical synthesis, DNA sequences encoding the proteins of the invention (or functional variants, derivatives, or analogs thereof). This DNA sequence can then be introduced into a variety of existing DNA molecules known in the art, such as vectors, and into cells. Mutations can be introduced into the protein sequence of the invention by methods such as mutation PCR or chemical synthesis.

The invention also provides a detection test kit comprising an optical probe or fusion polypeptide or polynucleotide described herein or an optical probe or fusion polypeptide prepared according to the method described herein. The kit also optionally contains additional reagents required for detecting lactate with the optical probe. The additional reagents are well known in the art.

The invention also relates to a nucleic acid construct containing the polynucleotides described herein, and one or more regulatory sequences operably linked to such sequences. The polynucleotides described herein can be manipulated in a variety of ways to guarantee expression of said polypeptide or protein. The nucleic acid construct can be manipulated according to the difference or requirements of the expression vector prior to insertion of the nucleic acid construct into the vector. Techniques that utilize recombinant DNA methods to alter polynucleotide sequences are known in the art.

In certain embodiments, the nucleic acid construct is a vector. The vector may be a cloning vector, an expression vector, or a homologous recombination vector. The polynucleotides of the present invention can be cloned into many types of vectors, e.g., plasmids, phagemids, phage derivatives, animal viruses, and cosmids. Cloning vectors can be used to provide coding sequences for proteins or polypeptides of the invention. Expression vectors can be provided to cells in the form of bacterial or viral vectors. Expression of the present polynucleotides is generally achieved by operably linking the polynucleotides of the invention to a promoter and incorporating the construct into an expression vector. This vector may be suitable for replicating and integrating eukaryotic cells. A typical expression vector contains an expression control sequence that can be used to regulate the expression of the desired nucleic acid sequence. In one or more embodiments, the cloning vector and the expression vector are one vector, i.e., a cloning expression vector. Homologous recombination vectors are used to integrate the expression frame described herein into the host genome.

The term "expression control sequence" used herein refers to elements that regulate the transcription, translation, and expression of a gene of interest and can be operably linked to the gene of interest, which may be a replication origin, promoter, marker gene, or translational control element, including enhancers, operons, terminators, ribosome binding sites, etc., and the choice of expression control sequence depends on the host cell used. In recombinant expression vectors, "operable ligation" refers to the attachment of the nucleotide sequence of interest to a regulatory sequence in a manner that allows the expression of the nucleotide sequence. Those skilled in the art are well known of methods for constructing expression vectors containing the present fusion protein coding sequences and suitable transcription/translation control signals. These include in vitro recombinant DNA techniques, DNA synthesis techniques, in vivo recombination techniques, etc. Said DNA sequence can be effectively ligated to an appropriate promoter in an expression vector to direct mRNA synthesis. Representative examples of these promoters are: the lac or trp promoter of *E. coli*; λ-phage PL promoter; eukaryotic promoters including the CMV immediate early promoter, the HSV thymidine kinase promoter, the early and late SV40 promoters, the LTR of retroviruses, and several other promoters known to control gene expression in prokaryotic or eukaryotic cells or their viruses. The expression vector also includes a ribosome binding site for translation initiation and a transcription terminator. In one embodiment, the expression vector may use a commercially available pET28a vector with no other special requirements. Exemplarily, BamHI and EcoRI were employed to double digest the nucleotide sequence encoding the optical probe and the expression vector, respectively, and then the enzymatic cleavage products of both were ligated to give recombinant expression vectors. The present invention makes no special limitation to the specific steps and parameters of enzymatic cleavage and ligation, and those conventional in the art would work.

After a recombinant expression vector is obtained, this vector is transformed into host cells to produce a protein or peptide including the fusion protein. This transfer process can be performed with conventional techniques well known to those skilled in the art, such as transformation or transfection. The host cells described herein refer to cells capable of receiving and accommodating recombinant DNA molecules, which are sites of recombinant gene amplification, and ideally the recipient cell should satisfy both conditions of easy access and proliferation. The "host cells" of the invention may include prokaryotic cells and eukaryotic cells, specifically including bacterial cells, yeast cells, insect cells, and mammalian cells. Specific examples can be bacterial cells of *E. coli, Streptomyces* spp., *Salmonella typhimurium,* fungal cells such as yeast, plant cells, insect cells of Drosophila S2 or Sf9, CHO, COS, HEK293, HeLa cells, or animal cells such as Bowes melanoma cells, among others, including but not limited to those host cells described above. The host cells are said to prefer a variety of cells favouring the expression or fermentative production of the gene product, such cells being well known and commonly used in the art. Those skilled in the art well know how to select appropriate vectors, promoters, enhancers, and host cells.

The methods of transfer to host cells described herein are conventional methods in the art, including calcium phosphate or calcium chloride coprecipitation, DEAE-mannan-mediated transfection, lipofection, naturally competent cells, chemically mediated transfer, or electroporation. When the host is a prokaryote such as E. *coli,* the process described is preferably CaCl₂ method or MgCl₂ method, and their steps used are well known in the art. When the host cell is a eukaryotic cell, the following DNA transfection methods are used: calcium phosphate coprecipitation, conventional mechanical methods such as microinjection, electroporation, liposome packaging, etc.

After transferring the expression vector into host cells, the host cells transferred with the expression vector are subjected to expansion and expression culture, and the lactate optical probe is isolated. The host cells are expanded for expression by conventional methods. Depending on the host cell species used, the medium used in culture may be various conventional media. Culture is performed under conditions suitable for host cell growth.

In the present invention, an optical probe is expressed intracellularly, on the cell membrane, or secreted outside the cell. Recombinant proteins can be isolated or purified by various separation methods, if desired, using their physical, chemical, and other properties. The present invention does not have a special limit to a process for separating said lactate fluorescent proteins, and a conventional method for the isolation of fusion proteins in the art would work. These methods are well known to those skilled in the art and include, but are not limited to: conventional renaturation treatments, salting out methods, centrifugation, osmotic disruption, sonication, ultra centrifugation, molecular sieving chromatography, adsorption chromatography, ion exchange chromatography, high performance liquid chromatography (HPLC) and other liquid chromatography techniques and the combination of these methods. In one embodiment, His tag affinity chromatography is utilized for optical probe isolation.

The invention also provides use of said lactate optical probes in real-time localization, quantitative detection of lactate as well as high-throughput compound screening. In one aspect, said lactate optical probe, preferably linked with signal peptides at different parts of the cell, is transferred into cells for real-time localization of lactate by detecting the strength of fluorescence signals in cells; corresponding quantitative detection of lactate is performed by a lactate standard titration curve. The lactate standard titration curve described herein is plotted from the fluorescence signals of a lactate optical probe in the presence of different concentrations of lactate. The lactate optical probe described herein is directly transferred into cells, which is more accurate without time-consuming sample processing process during the real-time localization and quantitative detection of lactate. The present lactate optical probe, when performing high-throughput compound screening, adds different compounds to the cell culture fluid, determines changes in lactate content, and thus screens out compounds that influence the changes in lactate content. The use of the lactate optical probes described herein in real-time localization, quantitative detection of lactate as well as high-throughput compound screening are all non-diagnostic and therapeutic purposes, not related to the diagnosis and treatment of diseases.

### Exemplary embodiments

1. An optical probe comprising a lactate-sensitive polypeptide and an optically active polypeptide, wherein the optically active polypeptide is located in the sequence of the lactate-sensitive polypeptide.
2. The optical probe according to embodiment 1, wherein the lactate-sensitive polypeptide has the sequence shown in SEQ ID No: 1 or a functional fragment thereof, or a sequence having at least 70% sequence identity with it,
   Preferably, the lactate-sensitive polypeptide is shown as amino acids 80-258 of SEQ ID No: 1 or shown in SEQ ID No: 1, or a sequence having at least 70% sequence identity with it,
   Preferably, the lactate-sensitive polypeptide comprises mutations at the following sites: (1) P189 and/or P190, and optionally (2) M185, more preferably, the mutations comprise: (1) P189R and P190D, P189R and P190A, P189R and P190I, P189R and P190Q, P189R and P190N, P189D and P190D, P189D and P190E, P189D and P190V, P189D and P190L, P189D and P190F, P189D and P190I, P189D and P190Q, P189D and P190N, P189D and P190G, P189D and P190Y, P189D and P190W, P189E and P190R, P189E and P190A, P189E and P190V, P189E and P190Q, P189A and P190L, P189A and P190F, P189A and P190M, P189A, P189A and P190N, P189A and P190G, P189A and P190H, P189A and P190T, P189V and P190D, P189V and P190E, P189V and P190A, P189V, P189V and P190N, P189V and P190H, P189V and P190Y, P189L and P190V, P189L and P190F, P189L and P190M, P189L and P190G, P189L and P190H, P189F and P190D, P189F and P190L, P189F and P190F, P189F and P190I, P189F and P190N, P189F and P190H, P189F and P190Y, P189F and P190K, P189F and P190T, P189F and P190W, P189I and P190R, P189I and P190D, P189I and P190A, P189I and P190V, P189I and P190M, P189I and P190Q, P189I and P190G, P189I and P190Y, P189I and P190S, P189I and P190T, P189M and P190R, P189M and P190D, P189M and P190E, P189M and P190F, P189M and P190G, P189M and P190S, P189M and P190W, P189C and P190D, P189C and P190E, P189C and P190F, P189C and P190I, P189C and P190M, P189C and P190C, P189C, P189C and P190H, P189C and P190Y, P189C and P190S, P189C and P190W, P190L, P190F, P190I, P190Q, P190N, P190K, P190T, P189Q and P190E, P189Q and P190A, P189Q and P190V, P189Q and P190M, P189Q and P190C, P189Q and P190Q, P189Q and P190H, P189Q and P190S, P189N and P190R, P189N and P190D, P189N and P190L, P189N and P190F, P189N and P190C, P189N, P189N and P190N, P189N and P190G, P189N and P190H, P189N and P190Y, P189N and P190T, P189G and P190V, P189G and P190F, P189G and P190M, P189G and P190C, P189G and P190G, P189G and P190H, P189G and P190K, P189G and P190W, P189H and P190R, P189H and P190D, P189H and P190E, P189H and P190L, P189H and P190S, P189Y and P190R, P189Y and P190L, P189Y and P190N, P189Y and P190H, P189Y and P190S, P189Y and P190T, P189K and P190D, P189K and P190E, P189K and P190V, P189K and P190L, P189K and P190F, P189K and P190I, P189K and P190M, P189K, P189K and P190Q, P189K and P190N, P189K and P190Y, P189K and P190K, P189K and P190T, P189S and P190E, P189S and P190A, P189S and P190L, P189S and P190F, P189S and P190M, P189S and P190C, P189S, P189S and P190Q, 189S and P190Y, P189S and P190K, P189S and P190S, P189T and P190R, P189T and P190D, P189T and P190M, P189T and P190C, P189T, P189T and P190Q, P189T and P190N, P189T and P190H, P189T and P190Y, P189T and P190K, P189T and P190W, P189W and P190A, P189W and P190V, P189W and P190F, P189W, P189W and P190Q, P189W and P190H, P189W and P190S, P189W and P190T, P189W and P190W and optionally (2) M185F, M185Y, M185L, M185I, M185Q, M185G, M185H, M185A, M185P, M185N, M185C, M185W, M185S, M185V, M185D, M185T, M185R, M185E or M185K.
3. The optical probe according to embodiments 1 or 2, wherein the optically active polypeptide is located in a position selected from the group consisting of: 93-97, 119-121, 137-141, 158-161, 185-191, 208-210 and/or 230-232; preferably, the optically active polypeptide is located at one or more sites of the lactate-sensitive polypeptides selected from the group consisting: 93/94, 93/95, 93/96, 93/97, 94/95, 94/96, 94/97, 95/96, 95/97, 96/97, 119/120, 119/121, 120/121, 137/138, 137/139, 137/140, 137/141, 138/139, 138/140, 138/141, 139/140, 139/141, 140/141, 158/159, 158/160, 158/161, 159/160, 159/161, 160/161, 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/191, 190/191, 208/209, 208/210, 209/210, 230/231, 230/232 and/or 231/232.
4. The optical probe according to embodiment 3, wherein the optically active polypeptide is located in residues 185-191 of the lactate-sensitive polypeptide, preferably, the present optical probe has or consists of the sequence shown in SEQ ID Nos: 6-30, 34-40.
5. A nucleic acid sequence selected from the group consisting of
   (1) a polynucleotide encoding the optical probe according to any one of embodiments 1-4;
   (2) a fragment of (1);
   (3) a complement of (1) or (2).
6. A nucleic acid construct comprising the nucleic acid sequence according to embodiment 5, preferably the nucleic acid construct is an expression vector.
7. A host cell, wherein the host cell
   (1) expresses the optical probe according to any one of embodiments 1-4;
   (2) comprises the nucleic acid sequence according to embodiment 5; or
   (3) comprises the nucleic acid construct according to embodiment 6,
   Preferably, the nucleic acid construct is an expression vector.
8. A method for producing the optical probe according to any one of embodiments 1-4, comprising culturing the host cells according to embodiment 7, and separating said optical probe from culture.
9. Use of the optical probe according to any one of embodiments 1-4, the nucleic acid sequence according to embodiment 5, the nucleic acid construct according to embodiment 6 in detection of lactate in a sample or compound screening, preferably the detection is lactate localization or quantitative detection.
10. A detection kit comprising
   (1) the optical probe according to any one of embodiments 1-4 or the optical probe prepared by the method according to embodiment 8;
   (2) the nucleic acid sequence according to embodiment 5;
   (3) the nucleic acid construct according to embodiment 6; or
   (4) the cell according to embodiment 7; and
   Additional reagents required for lactate detection using optical probes.

Concentrations, contents, percentages, and other values may be expressed with ranges available herein. It is also understood that use of these ranges is only for convenience and conciseness, which should be interpreted elastically to include values explicitly mentioned in the upper and lower limits of the range, but also to include all individual values or sub ranges included in the ranges.

### Example

The lactate optical probes provided by the invention are described in detail below in combination with the examples, but they cannot be understood as defining the scope of protection of the invention.

### I. Experimental materials and reagents

The conventional molecular biology cloning methods for genetic engineering and cell culture and imaging methods mainly used in examples are well known to those skilled in the art, for example, Roskams, J., Molecular Biology Laboratory Handbook*;* J. Sambrook, D. W. Russell ed, Molecular Cloning: A Laboratory Manual, translated by Peitang Huang et al. (3rd edition, August 2002, Science Press); R. I. Freshney et al., Culture of Animal Cells: a Manual of Basic Technique (5th edition), translated by Jingbo Zhang and Cunquan Xu; Juan S. Bonifacino, M. Daso, et al., Short Protocols in Cell Biology, translated by Jingbo Zhang *et al.*

The pCDFDuet-cpYFP based, pCDFDuet-lactate binding protein plasmid used in examples was constructed by the Protein Laboratory of East China University of Science and Technology, and the pCDFDuet plasmid vector was purchased from Novagen. All the primers used for PCR were synthesized, purified, and identified to be correct by mass spectrometry (MS) by Shanghai Generay Biotech Co., Ltd. The expression plasmids constructed in examples were all sequenced by BGI and Jie Li Sequencing. For each example, Taq DNA polymerase was purchased from Dongsheng Bio, pfu DNA polymerase was purchased from Tiangen Biochemical Technology (Beijing) Co., Ltd, primeSTAR DNA polymerase was purchased from Takara, and all three polymerases were purchased with corresponding polymerase buffer and dNTPs. Restriction enzymes such as BamHI, BglII, HindIII, NdeI, XhoI, EcoRI, SpeI, T4 ligase, and T4 phosphorylase (T4 PNK) were purchased from Fermentas with corresponding buffers and the like. Transfection reagent Lip2000 Kit was purchased from Invitrogen. Compounds such as lactate were purchased from Sigma. Chemical reagents such as inorganic salts were purchased from Sigma Aldrich unless specifically stated. HEPES salt, streptomycin sulfate, and puromycin were purchased from Ameresco. 96 well detection black plates, 384 well fluorescence detection black plates were purchased from Grenier.

The DNA purification kit used in examples was purchased from BBI (Canada), and the common Plasmid Mini Preparation Kit was purchased from Tiangen Biochemical Technology (Beijing) Co., Ltd. Clonal strain Mach1 was purchased from Invitrogen. Nickel column affinity chromatography columns and desalting column packing were from GE Healthcare.

The main instruments used in examples included: Biotek Synergy 2 multi-purpose microplate reader (Bio-Tek, USA), X-15R high-speed refrigerated centrifuge (Beckman, USA), Microfuge22R tabletop high-speed refrigerated centrifuge (Beckman, USA), PCR amplimer (Biometra, Germany), ultrasonic disruptor (Ningbo SCIENTZ), nucleic acid electrophoresis instrument (Shennengbocai company), spectrofluorometer (Varian, USA), CO₂ constant temperature cell culture incubator (SANYO), and inverted fluorescence microscope (Nikon, Japan).

### II. Molecular biology methods and cellular experimental methods

### II.1 Polymerase chain reaction (PCR):

1. PCR amplification of target fragments:
   This method was mainly used for gene fragment amplification and colony PCR to identify positive clones. The reaction system for PCR amplification was as follows: template sequence 0.5-1 µl, the forward primer (25 µM) 0.5 µl, the reverse primer (25 µM) 0.5 µl, 10×pfu buffer 5 µl, pfu DNA polymerase 0.5 µl, dNTP (10 mM) 1 µl, sterilized ultrapure water (ddH₂O) 41.5-42 µl, to a total volume of 50 µl. The PCR amplification program was as follows: denaturation at 95 °C for 2-10 min, 30 cycles (94-96 °C for 30-45 s, 50-65 °C for 30-45 s, 72 °C for a period (600 bp/min)), and extension at 72 °C for 10 min.
2. PCR amplification of long fragments (>2500bp):
   Long fragment amplification, primarily inverse PCR amplification vector, used in the invention is a technique used to obtain site directed mutations in examples below. Reverse PCR primers were designed at the variation site, where the 5 'end of one primer contained the variant nucleotide sequence. Amplified products contained the corresponding mutation site. The reaction system of amplification PCR of long fragments were as follows: template sequence (10 pg-1 ng) 1 µl, the forward primer (25 µM) 0.5 µl, the reverse primer (25 µM) 0.5 µl, 5×PrimerSTAR buffer 10 µl, PrimerSTAR DNA polymerase 0.5 µl, dNTPs (2.5 mM) 4 µl, sterilized ultrapure water (ddH₂O) 33.5 µl, to a total volume of 50 µl. The PCR amplification program was as follows: denaturation at 95 °C for 5 min, 30 cycles (98 °C for 10 s, 50-68 °C for 5-15 s, 72 °C for a period (1000 bp/min)), and extension at 72 °C for 10 min; or denaturation at 95 °C for 5 min, 30 cycles (98 °C for 10 s, 68 °C for a period (1000 bp/min)), and extension at 72 °C for 10 min.

### 11.2 Endonuclease digestion reaction:

The system for double digestion of plasmid vectors was as follows: plasmid vector 20 µl (approximately 1.5 µg), 10×Buffer 5 µl, restriction endonuclease 11-2 µl, restriction endonuclease 21-2 µl, made up to a total volume of 50 µl with sterilized ultrapure water. Reaction conditionsL 37 °C for 1-7 hours.

### 11.3 5' phosphorylation reaction of DNA fragments

Because the plasmid or genomic end extracted from microorganisms contains phosphate groups, but the PCR product does not, the 5' end base of the PCR product needs to be subjected to a phosphate group addition reaction, and only the DNA molecule with phosphate groups on its end allows ligation reaction. The phosphorylation reaction system was as follows: the PCR product fragments DNA sequences 5-8 µl, 10×T4 ligase buffer 1 µl, T4 polynucleotide kinase (T4 PNK) 1µl, sterilized ultrapure water 0-3 µl, to a total volume of 10 µl. The reaction conditions were 37 °C for 30 min-2 hours followed by inactivation at 72 °C for 20 min.

### 11.4 Ligation of target fragments and vectors

There are differences in the ligation method between different fragments and vectors, and three ligation methods are used in this invention.
1. Ligation of blunt end short fragments and linearized vectors
   The principle of this method is that the blunt end product obtained by PCR, after phosphorylation of the 5' end of the DNA fragment by T4 PNK, is ligated with a linearized vector in the presence of PEG4000 and T4 DNA ligase to obtain a recombinant plasmid. The homologous recombination ligation system was as follows: T4 PNK treated DNA fragment 4 µl, linearized vector fragment 4 µl, PEG4000 1 µl, 10×T4 ligase buffer 1 µl, T4 DNA ligase 1 µl, to total of 10 µl. The reaction condition was 22 °C for 30 min.
2. Ligation of DNA fragments containing cohesive ends and vector fragments containing cohesive ends
   DNA fragments cleaved by restriction enzymes often give rise to overhang sticky ends and can therefore be joined with sticky end vector fragments containing sequence complementarity to form recombinant plasmids. The ligation reaction system was as follows: digested PCR product fragments DNA 1-7 µl, digested plasmid 0.5-7 µl, 10×T4 ligase buffer 1 µl, T4 DNA ligase 1 µl, sterilized ultrapure water added to a total volume of 10 µl. The reaction condition was 16 °C for 4-8 hours.
3. Ligation reactions in which the products of DNA fragments phosphorylated at the 5' end were self circularized after site directed mutagenesis were introduced by inverse PCR
   The recombinant plasmid was generated by ligating the 3' and 5' ends of the linearized vector into a DNA fragment phosphorylated at the 5' end by a self circularization ligation reaction. The reaction system for self cyclization ligation was as follows: phosphorylation reaction system 10 µl, T4 ligase (5 U/µl) 0.5 µl, to a total volume of 10.5 µl. The reaction condition was 16°C for 4-16 hours.

### II.5 Preparation and transformation of competent cells

Preparation of competent cells:
1. A single colony (e.g., Mach1) is picked and inoculated in 5 ml LB medium at 37°C on a shaker overnight.
2. 0.5-1 ml of overnight cultured broth was taken and transferred into 50 ml LB medium and incubated at 37 °C at 220 rpm for 3 to 5 hours until the OD600 reached 0.5.
3. The cells were precooled in ice bath for 2 hours.
4. 4 °C, centrifugation at 4000 rpm for 10 min.
5. The supernatant was discarded, the cells were resuspended with 5 ml of precooled buffer, and resuspension buffer was added again after homogenization to a final volume of 50 ml.
6. Ice bath for 45 min.
7. 4 °C, centrifugation at 4000 rpm for 10 min, the bacteria were resuspended with 5 ml ice precooled storage buffer.
8. 100 µl of broth was loaded to each EP tube and frozen at -80 °C or in liquid nitrogen.

Resuspension buffer: CaCl₂ (100 mM), MgCl₂ (70 mM), NaAc (40 mM)
Storage buffer: 0.5 mL DMSO, 1.9 mL 80% glycol, 1 mL 10×CaCl₂ (1 M), 1 mL 10×MgCl₂ (700 mM), 1mL 10×NaAc (400 mM), 4.6 mL ddH₂O

### Transformation of compenent cells:

1. Take 100 µl competent cells were taken and thawed on an ice bath.
2. The appropriate volume of ligation product was added, mixed by gently pipetting, and incubated in ice bath for 30 min. The volume of ligation product normally added was less than 1/10 of the volume of competent cells.
3. The broth was put into a 42 °C water bath for heat shock for 90 s and quickly transferred to an ice bath for 5 min.
4. 500 µl LB was added and incubated at 200 rpm on a constant temperature shaker at 37 °C for 1 hour.
5. The broth was centrifuged at 4000 rpm for 3 min and 200 µl supernatant was added to mix the bacteria well and spread evenly on the surface of the agar plate containing appropriate antibiotics, and the plate is inverted in a 37 °C incubator overnight.

### II.6 Expression, purification, and fluorescent detection of proteins

1. The expression vectors were transformed into JM109 (DE3) cells, incubated inverted overnight, and the clones were picked from the plates into 250 ml Erlenmeyer flasks, placed in a shaker at 37 °C and incubated at 220 rpm to OD = 0.4-0.8, then 1/1000 (V/V) of IPTG (1 M) was added to induce expression at 18 °C for 24-36 hours.
2. Upon completion of inducible expression, cells were harvested by centrifugation at 4000 rpm for 30 min, resuspended in 50 mM phosphate buffer and disrupted ultrasonically until the broth were clear, centrifuged at 9600 rpm at 4 °C for 20 min.
3. The centrifuged supernatant was used to obtain protein by purification through a self packed nickel column affinity chromatography column, and the protein obtained after nickel column affinity chromatography was further used to obtain protein dissolved in 20 mM MOPS buffer (pH 7.4) or phosphate buffer PBS.
4. After purified proteins were subjected to SDS-PAGE, probes were diluted to a protein solution having a final concentration of 5-10 µM using assay buffer (100 mm HEPES, 100 mM NaCl, pH 7.3) or phosphate buffer PBS. Lactate was formulated as a stock solution at a final concentration of 1 M with assay buffer (20 mM MOPS, pH 7.4) or phosphate buffer PBS.
5. 100 µl 5 µM of protein solution was taken, incubated at 37 °C for 5 min, lactate was added to mix to a final concentration of 100 mM, respectively, the optical absorption of the protein at 340 nm was determined using a multifunctional fluorescence microplate reader.
6. 100 µl 1 µM of protein solution was taken, incubated at 37 °C for 5 min, lactate was added for titration, and the fluorescence intensity of 528 nm emission after 485 nm fluorescence excitation of the protein was measured. The fluorescence excitation and emission determination of the samples were done using a multifunctional fluorescence microplate reader.
7. 100 µl 1 µM of protein solution was taken, incubated at 37 °C for 5 min, and lactate was added to determine the absorption and fluorescence spectra of the proteins. Determination of the absorption and fluorescence spectra of the samples was done by spectrophotometer and spectrofluorometer.

### II.7 Transfection of mammalian cells and detection of fluorescence

1. pCDNA3. 1+-based lactate optical probe plasmids were transfected into HeLa by using the transfection reagent Lipofectamine2000 (Invitrogen) and incubated in a cell incubator at 37 °C with 5% CO₂. After the exogenous gene was fully expressed for 24 ~ 36 hours, the fluorescence detection was performed.
2. After induction of expression, adherent HeLa cells were washed three times with PBS and placed in HBSS solution for fluorescence microscopy and microplate reader assays, respectively.

### Example 1. Lactate binding protein plasmid

The LldR (80-258) gene in the *E. coli* gene was amplified by PCR and digested with BamHI and EcoRI after gel electrophoresis of the PCR products, while the corresponding double digestion of the pCDFDuet1 vector was performed. After ligation with T4 DNA ligase, MachI was transformed with the product and the transformed MachI was spread onto LB plates (50 ug/ ml streptomycin sulfate) and placed in culture at 37 °C overnight. After growing MachI transformants were subjected to plasmid extraction, they were subjected to PCR. Positive plasmids were sequenced to be correct for subsequent plasmid construction.

### Example 2. Expression and detection of optical probes with cpYFP inserted at different sites

In this example, the insertion of cpYFP at the following sites was selected according to the crystal structure of the lactate binding protein based on pCDFDuet-LldR (80-258) to obtain corresponding pCDFDuet-LldR(80-258)-cpYFP plasmid: 93/94, 93/95, 93/96, 93/97, 94/95, 94/96, 94/97, 95/96, 95/97, 96/97, 119/120, 119/121, 120/121, 137/138, 137/139, 137/140, 137/141, 138/139, 138/140, 138/141, 139/140, 139/141, 140/141, 158/159, 158/160, 158/161, 159/160, 159/161, 160/161, 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/191, 190/191, 208/209, 208/210, 209/210, 230/231, 230/232 or 231/232. The amino acid sequences of exemplary optical probes are shown in Table 1.

**Table 1. Sequences of optical probes**

| Sequence | Insertion site |
|---|---|
| SEQ ID NO:6 | 185/186 |
| SEQ ID NO:7 | 185/187 |
| SEQ ID NO:8 | 185/188 |
| SEQ ID NO:9 | 185/189 |
| SEQ ID NO:10 | 185/190 |
| SEQ ID NO:11 | 186/187 |
| SEQ ID NO:12 | 186/188 |
| SEQ ID NO:13 | 186/189 |
| SEQ ID NO:14 | 186/190 |
| SEQ ID NO:15 | 187/189 |
| SEQ ID NO:16 | 189/191 |
| SEQ ID NO:17 | 190/191 |

DNA fragments of cpYFP were generated by PCR while the pCDFDuet-LldR (80-258) linearized vector containing different break sites was generated by reverse PCR amplification, the linearized pCDFDuet-LldR (80-258) and cpYFP fragments were ligated under the action of homologous recombinases to generate recombinant plasmids, which were selected by colony PCR, and the positive clones were sequenced by Shanghai Jie Li Biotechnology Co., Ltd.

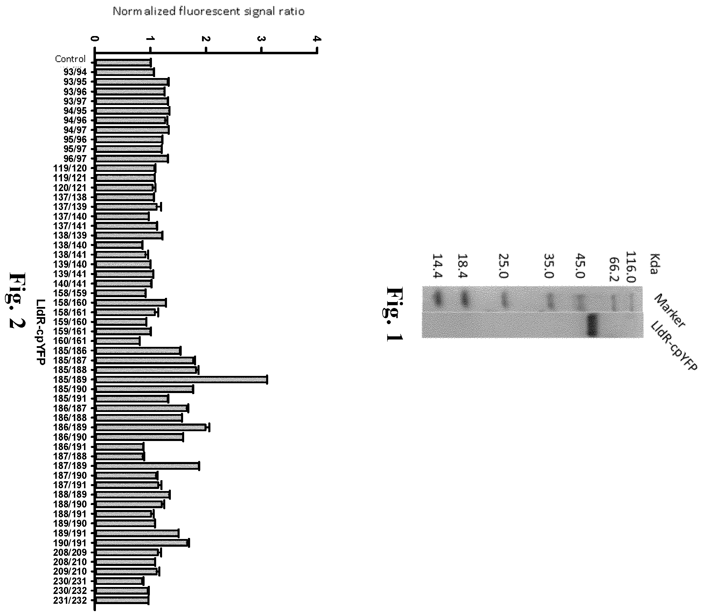

After sequenced to be correct, the recombinant plasmids were transformed into BL21 (DE3) to induce expression, and proteins were purified, electrophoretically sized around 48 kDa by SDS-PAGE. This size was in accordance with the size of the His-tag purification tag containing LldR (80-258)-cpYFP fusion protein expressed by pCDFDuet-LldR (80-258)-cpYFP. The results were shown in Fig. 1.

Purified L1dR (80-258)-cpYFP fusion protein and the control protein cpYFP were subjected to lactate responsive detection, dividing the detection signal of the fusion fluorescent protein containing 10 mM lactate by that of the fusion fluorescent protein without lactate. Results were shown in Fig. 2, the detection results showed that optical probes that responded more than 1.5-fold to lactate had optical probes having insertions at 185/186, 185/187, 185/188, 185/189, 185/190, 186/187, 186/188, 186/189, 186/190, 187/189, 189/191 and 190/191 sites or corresponding amino acid sites of their family proteins.

### Example 3. Expression and detection of cpGFP optical probes with different insertion sites

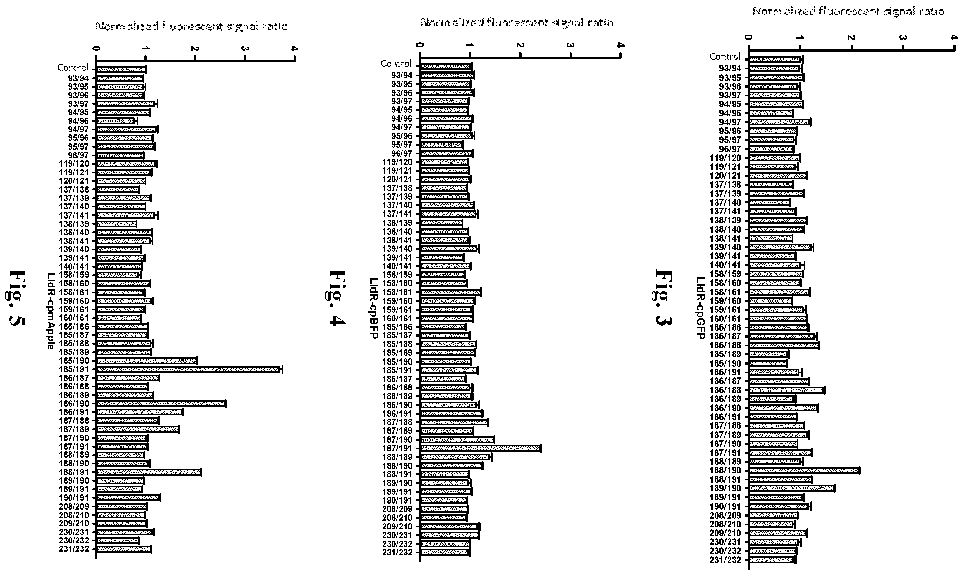

The lactate green fluorescent protein fluorescent probe was constructed by replacing cpYFP with cpGFP as in Example 2, expressed and detected as in Example 2. As shown in Fig. 3, the detection results showed that the optical probe that responded to lactate more than 1.5 times had insertions at 188/190, and 189/190 sites.

### Example 4. Expression and detection of optical probes having cpBFP inserted at different sites

The lactate blue fluorescent protein fluorescent probe was constructed by replacing cpYFP with cpBFP according to the procedure in Example 2, expressed and detected as in Example 2. As shown in Fig. 4, the detection results showed that the optical probes that responded to lactate more than 1.5-fold had insertions at 187/190, and 187/191 sites.

### Example 5. Expression and detection of optical probes having cpmApple inserted at different sites

A lactate red fluorescent protein fluorescent probe was constructed by replacing cpYFP with cpmApple as in Example 2, expressed and detected as in Example 2. As shown in Fig. 5, the detection results showed that the optical probe that responded more than 1.5-fold to lactate had insertions at 185/190, 185/191, 186/190, 186/191, 187/189 and 188/191 sites.

### Example 6. Expression and detection of an optical probe double site mutant

Mutants of the two sites, 189 and 190, were constructed on the basis of LldR(80-258)-185/189-cpYFP. Plasmid pCDFDuet-LldR(80-258)-185/189-cpYFP was linearized by reverse PCR, mutations were introduced at mutation sites by primers, and the obtained PCR products was ligated by adding phosphorus with PNK, T4 DNA ligase, and PEG4000, transformed into BL21 (DE3), and then screened. The detection signal for fusion protein and control protein cpYFP with 10 mM lactate was divided by the detection signal without lactate, and the response of the respective mutants to lactate was shown in Table 2. Among these samples, those that corresponded to lactate for more than 2-fold samples were P189/P190(WT), P189S(12H4), P189C/P190D(14B5), P189C/P190Y(14D2), P189N/P190Y(3D1), P189R/P190I(14H5), P189M/P190D(10F4), P189H/P190R(13H6), P189N(2F5), P189F/P190D(3C2), P189F/P190H(2G3), P189N/P190F(9B6), P189C/P190F(2A5) and P189H/P190D(1A3). Sequences of exemplary optical probe mutants were shown in Table 3. Exemplary nucleic acid sequences were shown in SEQ ID No: 31 (L1dR(80-258)-185/189-P189F/P190D-cpYFP).

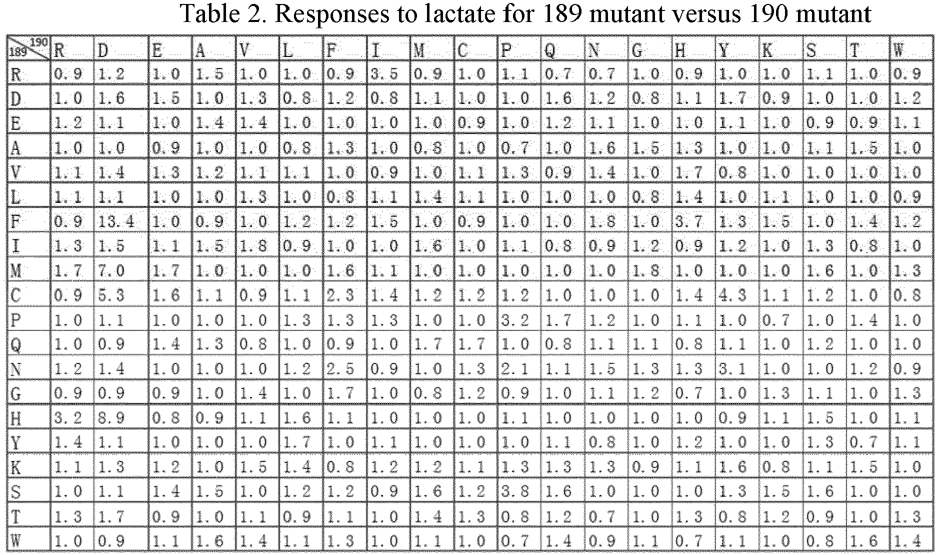

**Table. 3**

| Sequences | Mutation sites |
|---|---|
| SEQ ID NO:18 | P189N |
| SEQ ID NO:19 | P189S |
| SEQ ID NO:20 | P189C/P190F |
| SEQ ID NO:21 | P189N/P190F |
| SEQ ID NO:22 | P189N/P190Y |
| SEQ ID NO:23 | P189H/P190R |
| SEQ ID NO:24 | P189R/P190I |
| SEQ ID NO:25 | P189F/P190H |
| SEQ ID NO:26 | P189C/P190Y |
| SEQ ID NO:27 | P189C/P190D |
| SEQ ID NO:28 | P189M/P190D |
| SEQ ID NO:29 | P189H/P190D |
| SEQ ID NO:30 | P189F/P190D |

### Example 7. Titration curve of the optical probe of the two-site mutant

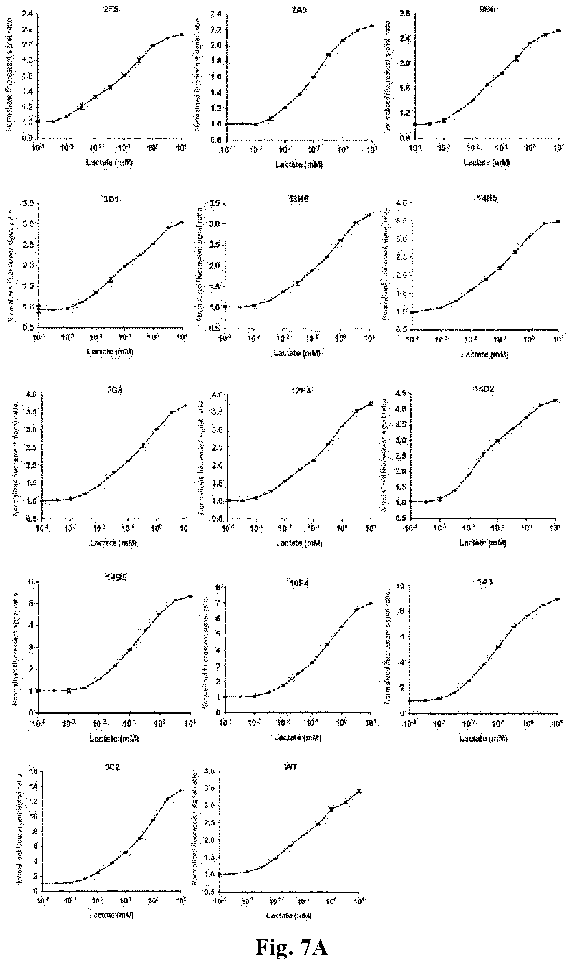

The optical probes that responded more than 2 fold to lactate obtained in Example 6, i.e., P189/P190(WT), P189N(2F5), P189C/P190F(2A5), P189N/P190F(9B6), P189N/P190Y(3D1), P189H/P190R(13H6), P189R/P190I(14H5), P189F/P190H(2G3), P189S(12H4), P189C/P190Y(14D2), P189C/P190D(14B5), P189M/P190D(10F4), P189H/P190D(1A3) and P189F/P190D(3C2) was subjected to detection with lactate in concentration gradient, changes in the ratio of fluorescence intensity at 420 nm excitation and 528 nm emission to that of 485 nm excitation and 528 nm emission were detected. The Kd (binding constant) of wild-type WT and mutants 2F5, 2A5, 9B6, 3D1, 13H6, 14H5, 2G3, 12H4, 14D2, 14B5, 10F4, 1A3 and 3C2 were 112 µM, 67 µM, 114 µM, 46 µM, 95 µM, 194 µM, 70 µM, 152 µM, 88 µM, 41 µM, 57 µM, 234 µM, 100 µM, and 95 µM, respectively, and their magnitudes of change were 3.4 fold, 2.1 fold, 2.3 fold, 2.5 fold, 3.1 fold, 3.2 fold, 3.5 fold, 3.7 fold, 3.8 fold, 4.3 fold, 5.3 fold, 7.0 fold, 8.9 fold and 13.4 fold, respectively, and the results were shown in Fig. 7A.

### Example 8. Expression and detection of an optical probe of the three-site mutant

For the two-site mutants based on 189 and 190 in Example 7, the optical probes that responded more than 5-fold to lactate were P189C/P190D(14B5), P189M/P190D(10F4), P189F/P190D(3C2) and P189H/P190D(1A3). Saturation mutations at M185 site were made on the basis of these four mutants, and the lactate responsive profiles of all mutants were shown in Table 4. The samples that responded more than 2-fold to lactate were M185F/P189F/P190D, M185I/P189F/P190D, M185G/P189F/P190D, M185H/P189F/P190D, M185A/P189F/P190D, M185S/P189F/P190D, M185V/P189F/P190D, M185F/P189H/P190D, M185Y/P189H/P190D, M185L/P189H/P190D, M185I/P189H/P190D, M185G/P189H/P190D, M185Q/P189H/P190D, M185N/P189H/P190D, M185C/P189H/P190D, M185W/P189H/P190D, M185S/P189H/P190D, M185V/P189H/P190D, M185D/P189H/P190D, M185T/P189H/P190D, M185E/P189H/P190D, M185F/P189M/P190D, M185Y/P189M/P190D, M185L/P189M/P190D, M185I/P189M/P190D, M185G/P189M/P190D, M185Q/P189M/P190D, M185H/P189M/P190D, M185A/P189M/P190D, M185C/P189M/P190D, M185W/P189M/P190D, M185S/P189M/P190D, M185V/P189M/P190D, M185T/P189M/P190D, M185E/P189M/P190D, M185F/P189C/P190D, M185Y/P189C/P190D, M185L/P189C/P190D, M185I/P189C/P190D, M185G/P189C/P190D, M185Q/P189C/P190D, M185H/P189C/P190D, M185A/P189C/P190D, M185P/P189C/P190D, M185N/P189C/P190D, M185C/P189C/P190D, M185W/P189C/P190D, M185S/P189C/P190D, M185V/P189C/P190D, M185D/P189C/P190D, M185T/P189C/P190D, M185E/P189C/P190D, M185K/P189C/P190D. The samples that responded more than 5-fold to lactate were: M185F/P189H/P190D, M185L/P189H/P190D, M185I/P189H/P190D, M185S/P189H/P190D, M185V/P189H/P190D, M185L/P189M/P190D, M185A/P189M/P190D.

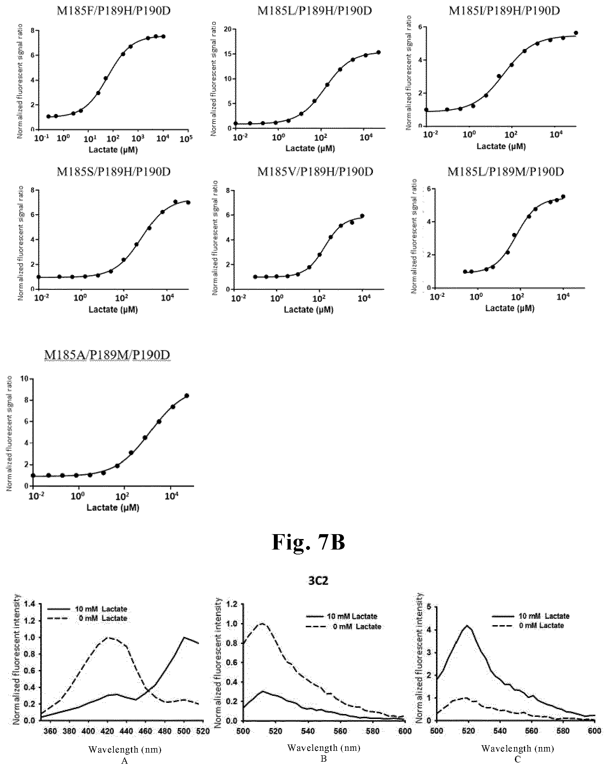

The sequences of exemplary optical probe mutants were shown in Table 5. Lactate assays with concentration gradients were performed on these nine samples to detect changes in the ratio of fluorescence intensity at 420 nm excition and 528 nm emission and that at 485 nm excition and 528 nm emission. The K_{d} (binding constant) values for M185F/P189H/P190D, M185L/P189H/P190D(G9), M185I/P189H/P190D, M185S/P189H/P190D, M185V/P189H/P190D, M185L/P189M/P190D, M185A/P189M/P190D were 61 µM, 154 µM, 42 µM, 660 µM, 210 µM, 62 µM, 1000 µM, respectively, their magnitudes of change were 8-fold, 15 fold, 5.6-fold, 7-fold, 6.2-fold, 5.5-fold, 8.4-fold, respectively, and the results were shown in Fig. 7B.

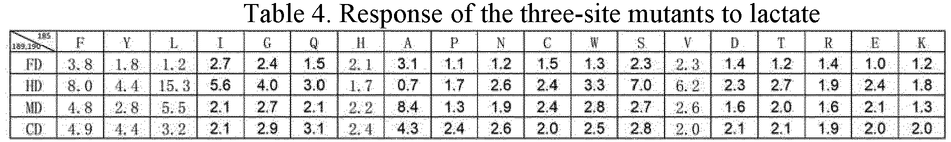

**Table 5**

| Sequences | Mutation sites |
|---|---|
| SEQ ID NO:34 | M185F/P189H/P190D |
| SEQ ID NO:35 | M185L/P189H/P190D |
| SEQ ID NO:36 | M185I/P189H/P190D |
| SEQ ID NO:37 | M185S/P189H/P190D |
| SEQ ID NO:38 | M185V/P189H/P190D |
| SEQ ID NO:39 | M 185L/P 189M/P 190D |
| SEQ ID NO:40 | M185A/P189M/P190D |

### Example 9. Spectral properties and specificity of the optical probes

Exemplarily, detection of fluorescence spectra was performed using a fluorescence spectrophotometer after subjecting purified lactate optical probes 3C2, 10F4, and G9 to 0 mM and 10 mM lactate treatments for 10 min, respectively.

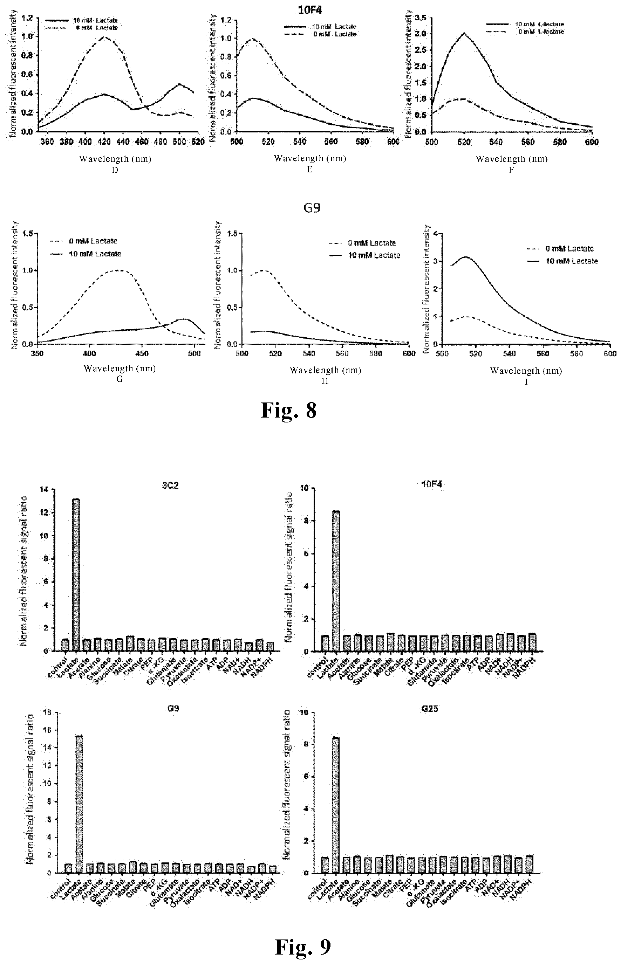

Determination of excitation spectra: excitation spectra were recorded with an excitation range of 350 nm to 515 nm and an emission wavelength of 530 nm, read every 5 nm. The results showed two excitation peaks at about 420 and 500 nm for probes 3C2, 10F4 and G9, as shown in Figs. 8A, 8D and 8G.

Determination of the emission spectra: the excitation wavelengths were fixed at 420 nm and 490 nm, respectively, and the emission spectra from 500 to 600 nm were recorded and read every 5 nm. The results showed that the fluorescence intensity of probe 3C2 after the addition of 10 mM lactate decreased at 420 nm excitation to 0.31 times that of the addition of 0 mM lactate; Tte fluorescence intensity enhancement at 490 nm excitation was 4.2-fold of that with the addition of 0 mM lactate, as shown in Figs. 8B and 8C. The fluorescence intensity of probe 10F4 at 420 nm excitation decreased to 0.37-fold of that with the addition of 0 mM lactate; the fluorescence intensity enhancement at 490 nm excitation was 3 times that with the addition of 0 mM lactate, as shown in Figs. 8E and 8F. Probe G9 showed a decrease in fluorescence intensity at 420 nm excitation after the addition of 10 mM lactate to 0.18 times that with the addition of 0 mM lactate; the fluorescence intensity enhancement at 490 nm excitation was 2.8 times that with the addition of 0 mM lactate, as shown in Figs. 8H and 8I.

The specificity was determined for the purified lactate optical probes 3C2, 10F4, G9 and G25, and the results showed that the probes were very specific, as shown in Fig. 9.

### Example 10. Subcellular organelle localization of optical probes

In this example, different localization signal peptides were used to fuse to an optical probe which was localized in different organelles.

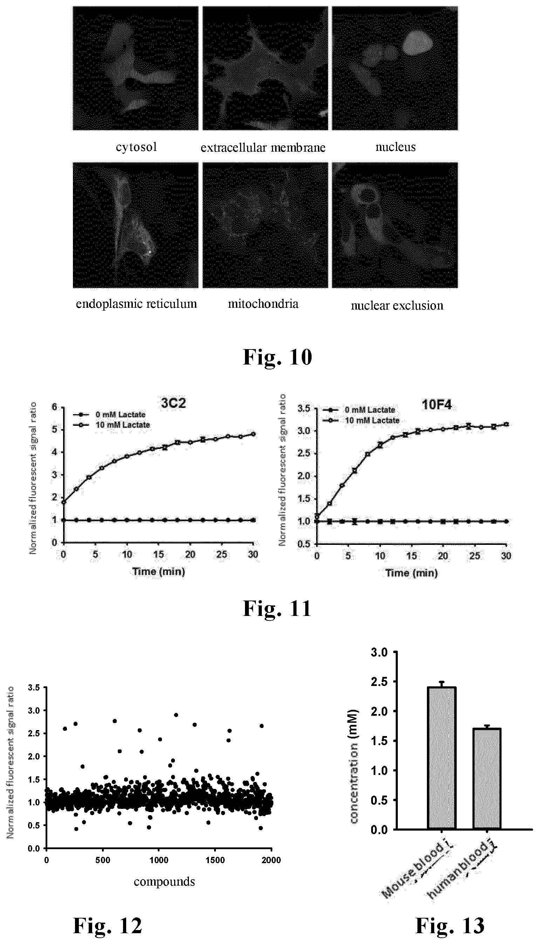

36 hours after transfection of HeLa cells with optical probe plasmids fused to different localization signal peptides, the cells were rinsed using PBS, placed in HBSS solution for fluorescence detection under the FITC channel using an inverted fluorescence microscope. The results were shown in Fig. 10. Lactate optical probes were able to localize to subcellular organelles including cytosol, extracellular membrane, nucleus, endoplasmic reticulum, mitochondria, nuclear exclusion, etc., by fusing with different specific localization signal peptides. Fluorescence was present in different subcellular structures, and the distribution and intensity of fluorescence varied.

### Example 11. Dynamic monitoring of lactate transmembrane transport

36 hours after transfection of HeLa cells with plasmids encoding cytosolically expressed optical probes, cells were rinsed using PBS, placed in HBSS solution, and then added with 10 mM lactate to detect changes in the ratio of the fluorescence intensity at 420 nm excitation and 528 nm emission to that of 485 nm excitation and 528 nm emission over 30 min. Results were shown in Fig. 11, the 485/420 of the samples with lactate addition gradually increased and could reach 4.8-fold and 3.1-fold maximum for 3C2 and 10F4, respectively, while the 485/420 for the control group without lactate addition was 1 and remained unchanged.

### Example 12. High throughput compound screening based optical probes in living cells

In this example, we used HeLa cells with cytosolic expression of lactate probe 3C2 for high-throughput compound screening.

Transfected HeLa cells were rinsed using PBS, placed in HBSS solution (without lactate) for 1 hour and then treated with 10 µM of the compound for 1 hour. Lactate was added dropwise in each sample, respectively. Changes in the ratio of fluorescence intensity at 420 nm excitation and 528 nm emission to that of 485 nm excitation and 528 nm emission were recorded using a microplate reader. Samples that were not treated with any compounds were used as controls for normalization. The results were shown in Fig. 12. Of the 2000 compounds used, the vast majority had minimal effects on lactate's entry into cells. Twelve compounds were able to increase the cellular uptake of lactate, and other six compounds were able to decrease the cellular uptake of lactate significantly.

### Example 13. Quantitative detection of lactate in blood with optical probes

In this example, we used purified lactate probe 3C2 for the analysis of lactate in blood supernatants of mice and humans.

After 10 min of treatment by mixing lactate probe 3C2 with diluted blood supernatants, the ratio of fluorescence intensity at 420 nm excition and 528 nm emission to that of 485 nm excition and 528 nm emission was measured using a microplate reader. Results were shown in Fig. 13, lactate levels were around 2.4 mM in mouse blood and 1.7 mM in human blood.

It is known from the above examples that the lactate optical probes provided in the invention had relatively small protein molecular weight and was prone to maturation, had large dynamic change in fluorescence, good specificity, and ability to be expressed in cells by gene manipulation methods, which could locate, quantitatively detect lactate in real time inside and outside cells; and enables high-throughput compound screening.

### Other embodiments

The instant specification describes several embodiments. It is understood, however, that the various improvements learned by those skilled in the art not to deviate from the conception and scope of the invention by reading these instructions shall also be included within the scope of the attached claims.

## Claims

1. An optical probe comprising a lactate-sensitive polypeptide and an optically active polypeptide, wherein the optically active polypeptide is located in the sequence of the lactate-sensitive polypeptide.

2. The optical probe according to claim 1, wherein the lactate-sensitive polypeptide has the sequence shown in SEQ ID No: 1 or a functional fragment thereof, or a sequence having at least 70% sequence identity with it,
preferably, the lactate-sensitive polypeptide is shown as amino acids 80-258 of SEQ ID No: 1 or shown as SEQ ID No: 1, or a sequence having at least 70% sequence identity with it,
preferably, the lactate-sensitive polypeptide comprises mutations at the following sites: (1) P189 and/or P190, and optionally (2) M185, more preferably, the mutations comprise: (1) P189R and P190D, P189R and P190A, P189R and P190I, P189R and P190Q, P189R and P190N, P189D and P190D, P189D and P190E, P189D and P190V, P189D and P190L, P189D and P190F, P189D and P190I, P189D and P190Q, P189D and P190N, P189D and P190G, P189D and P190Y, P189D and P190W, P189E and P190R, P189E and P190A, P189E and P190V, P189E and P190Q, P189A and P190L, P189A and P190F, P189A and P190M, P189A, P189A and P190N, P189A and P190G, P189A and P190H, P189A and P190T, P189V and P190D, P189V and P190E, P189V and P190A, P189V, P189V and P190N, P189V and P190H, P189V and P190Y, P189L and P190V, P189L and P190F, P189L and P190M, P189L and P190G, P189L and P190H, P189F and P190D, P189F and P190L, P189F and P190F, P189F and P190I, P189F and P190N, P189F and P190H, P189F and P190Y, P189F and P190K, P189F and P190T, P189F and P190W, P189I and P190R, P189I and P190D, P189I and P190A, P189I and P190V, P189I and P190M, P189I and P190Q, P189I and P190G, P189I and P190Y, P189I and P190S, P189I and P190T, P189M and P190R, P189M and P190D, P189M and P190E, P189M and P190F, P189M and P190G, P189M and P190S, P189M and P190W, P189C and P190D, P189C and P190E, P189C and P190F, P189C and P190I, P189C and P190M, P189C and P190C, P189C, P189C and P190H, P189C and P190Y, P189C and P190S, P189C and P190W, P190L, P190F, P190I, P190Q, P190N, P190K, P190T, P189Q and P190E, P189Q and P190A, P189Q and P190V, P189Q and P190M, P189Q and P190C, P189Q and P190Q, P189Q and P190H, P189Q and P190S, P189N and P190R, P189N and P190D, P189N and P190L, P189N and P190F, P189N and P190C, P189N, P189N and P190N, P189N and P190G, P189N and P190H, P189N and P190Y, P189N and P190T, P189G and P190V, P189G and P190F, P189G and P190M, P189G and P190C, P189G and P190G, P189G and P190H, P189G and P190K, P189G and P190W, P189H and P190R, P189H and P190D, P189H and P190E, P189H and P190L, P189H and P190S, P189Y and P190R, P189Y and P190L, P189Y and P190N, P189Y and P190H, P189Y and P190S, P189Y and P190T, P189K and P190D, P189K and P190E, P189K and P190V, P189K and P190L, P189K and P190F, P189K and P190I, P189K and P190M, P189K, P189K and P190Q, P189K and P190N, P189K and P190Y, P189K and P190K, P189K and P190T, P189S and P190E, P189S and P190A, P189S and P190L, P189S and P190F, P189S and P190M, P189S and P190C, P189S, P189S and P190Q, 189S and P190Y, P189S and P190K, P189S and P190S, P189T and P190R, P189T and P190D, P189T and P190M, P189T and P190C, P189T, P189T and P190Q, P189T and P190N, P189T and P190H, P189T and P190Y, P189T and P190K, P189T and P190W, P189W and P190A, P189W and P190V, P189W and P190F, P189W, P189W and P190Q, P189W and P190H, P189W and P190S, P189W and P190T, P189W and P190W and optionally (2) M185F, M185Y, M185L, M185I, M185Q, M185G, M185H, M185A, M185P, M185N, M185C, M185W, M185S, M185V, M185D, M185T, M185R, M185E or M185K.

3. The optical probe according to claims 1 or 2, wherein the optically active polypeptide is located in a position selected from the group consisting of: 93-97, 119-121, 137-141, 158-161, 185-191, 208-210 and/or 230-232; preferably, the optically active polypeptide is located at one or more sites of the lactate-sensitive polypeptides selected from the group consisting: 93/94, 93/95, 93/96, 93/97, 94/95, 94/96, 94/97, 95/96, 95/97, 96/97, 119/120, 119/121, 120/121, 137/138, 137/139, 137/140, 137/141, 138/139, 138/140, 138/141, 139/140, 139/141, 140/141, 158/159, 158/160, 158/161, 159/160, 159/161, 160/161, 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/191, 190/191, 208/209, 208/210, 209/210, 230/231, 230/232 and/or 231/232.

4. The optical probe according to claim 3, wherein the optically active polypeptide is located in residues 185-191 of the lactate-sensitive polypeptide, preferably, the present optical probe has or consists of the sequence shown in any of SEQ ID Nos: 6-30, and 34-40.

5. A nucleic acid sequence selected from the group consisting of
(1) a polynucleotide encoding the optical probe according to any one of claims 1-4;
(2) a fragment of (1);
(3) a complement of (1) or (2).

6. A nucleic acid construct comprising the nucleic acid sequence according to claim 5, preferably the nucleic acid construct is an expression vector.

7. A host cell, wherein the host cell
(1) expresses the optical probe according to any one of claims 1-4;
(2) comprises the nucleic acid sequence according to claim 5; or
(3) comprises the nucleic acid construct according to claim 6,
preferably, the nucleic acid construct is an expression vector.

8. A method for producing the optical probe according to any one of claims 1-4, comprising culturing the host cells according to claim 7, and separating said optical probe from the culture.

9. Use of the optical probe according to any one of claims 1-4, the nucleic acid sequence according to claim 5, the nucleic acid construct according to claim 6 in detection of lactate in a sample or in compound screening, preferably the detection is localization or quantitative detection of lactate.

10. A detection kit comprising:
(1) the optical probe according to any one of claims 1-4 or the optical probe prepared by the method according to claim 8;
(2) the nucleic acid sequence according to claim 5;
(3) the nucleic acid construct according to claim 6; or
(4) the cell according to claim 7; and
additional reagents required for lactate detection by optical probes.


SEQUENCE LISTING

<110>  East China University of Science and Technology

<120>  Lactic Acid Optical Probe, Preparation Method Thereof and
       Application Thereof

<130>  20A578

<150>  CN201911318003.9
<151>  2019-12-19

<160>  40

<170>  PatentIn version 3.5

<210>  1
<211>  258
<212>  PRT
<213>  Escherichia coli

<400>  1

Met Ile Val Leu Pro Arg Arg Leu Ser Asp Glu Val Ala Asp Arg Val
1               5                   10                  15

Arg Ala Leu Ile Asp Glu Lys Asn Leu Glu Ala Gly Met Lys Leu Pro
            20                  25                  30

Ala Glu Arg Gln Leu Ala Met Gln Leu Gly Val Ser Arg Asn Ser Leu
        35                  40                  45

Arg Glu Ala Leu Ala Lys Leu Val Ser Glu Gly Val Leu Leu Ser Arg
    50                  55                  60

Arg Gly Gly Gly Thr Phe Ile Arg Trp Arg His Asp Thr Trp Ser Glu
65                  70                  75                  80

Gln Asn Ile Val Gln Pro Leu Lys Thr Leu Met Ala Asp Asp Pro Asp
                85                  90                  95

Tyr Ser Phe Asp Ile Leu Glu Ala Arg Tyr Ala Ile Glu Ala Ser Thr
            100                 105                 110

Ala Trp His Ala Ala Met Arg Ala Thr Pro Gly Asp Lys Glu Lys Ile
        115                 120                 125

Gln Leu Cys Phe Glu Ala Thr Leu Ser Glu Asp Pro Asp Ile Ala Ser
    130                 135                 140

Gln Ala Asp Val Arg Phe His Leu Ala Ile Ala Glu Ala Ser His Asn
145                 150                 155                 160

Ile Val Leu Leu Gln Thr Met Arg Gly Phe Phe Asp Val Leu Gln Ser
                165                 170                 175

Ser Val Lys His Ser Arg Gln Arg Met Tyr Leu Val Pro Pro Val Phe
            180                 185                 190

Ser Gln Leu Thr Glu Gln His Gln Ala Val Ile Asp Ala Ile Phe Ala
        195                 200                 205

Gly Asp Ala Asp Gly Ala Arg Lys Ala Met Met Ala His Leu Ser Phe
    210                 215                 220

Val His Thr Thr Met Lys Arg Phe Asp Glu Asp Gln Ala Arg His Ala
225                 230                 235                 240

Arg Ile Thr Arg Leu Pro Gly Glu His Asn Glu His Ser Arg Glu Lys
                245                 250                 255

Asn Ala

<210>  2
<211>  246
<212>  PRT
<213>  Artificial Sequence

<220>
<223>  cpYFP

<400>  2

Tyr Asn Ser Asp Asn Val Tyr Ile Met Ala Asp Lys Gln Lys Asn Gly
1               5                   10                  15

Ile Lys Ala Asn Phe Lys Ile Arg His Asn Val Glu Asp Gly Ser Val
            20                  25                  30

Gln Leu Ala Asp His Tyr Gln Gln Asn Thr Pro Ile Gly Asp Gly Pro
        35                  40                  45

Val Leu Leu Pro Asp Asn His Tyr Leu Ser Phe Gln Ser Val Leu Ser
    50                  55                  60

Lys Asp Pro Asn Glu Lys Arg Asp His Met Val Leu Leu Glu Phe Val
65                  70                  75                  80

Thr Ala Ala Gly Ile Thr Leu Gly Met Asp Glu Leu Tyr Asn Val Asp
                85                  90                  95

Gly Gly Ser Gly Gly Thr Gly Ser Lys Gly Glu Glu Leu Phe Thr Gly

            100                 105                 110

Val Val Pro Ile Leu Val Glu Leu Asp Gly Asp Val Asn Gly His Lys
        115                 120                 125

Phe Ser Val Ser Gly Glu Gly Glu Gly Asp Ala Thr Tyr Gly Lys Leu
    130                 135                 140

Thr Leu Lys Leu Ile Cys Thr Thr Gly Lys Leu Pro Val Pro Trp Pro
145                 150                 155                 160

Thr Leu Val Thr Thr Leu Gly Tyr Gly Leu Lys Cys Phe Ala Arg Tyr
                165                 170                 175

Pro Asp His Met Lys Gln His Asp Phe Phe Lys Ser Ala Met Pro Glu
            180                 185                 190

Gly Tyr Val Gln Glu Arg Thr Ile Phe Phe Lys Asp Asp Gly Asn Tyr
        195                 200                 205

Lys Thr Arg Ala Glu Val Lys Phe Glu Gly Asp Thr Leu Val Asn Arg
    210                 215                 220

Ile Glu Leu Lys Gly Ile Asp Phe Lys Glu Asp Gly Asn Ile Leu Gly
225                 230                 235                 240

His Lys Leu Glu Tyr Asn
                245

<210> 3
<211> 241
<212> PRT
<213> Artificial Sequence

<220>
<223> cpGFP

<400> 3

Asn Val Tyr Ile Lys Ala Asp Lys Gln Lys Asn Gly Ile Lys Ala Asn
1               5                   10                  15

Phe Lys Ile Arg His Asn Ile Glu Asp Gly Gly Val Gln Leu Ala Tyr
            20                  25                  30

His Tyr Gln Gln Asn Thr Pro Ile Gly Asp Gly Pro Val Leu Leu Pro
        35                  40                  45

Asp Asn His Tyr Leu Ser Val Gln Ser Ile Leu Ser Lys Asp Pro Asn
    50                  55                  60

Glu Lys Arg Asp His Met Val Leu Leu Glu Phe Val Thr Ala Ala Gly
65                  70                  75                  80

Ile Thr Leu Gly Met Asp Glu Leu Tyr Lys Gly Gly Thr Gly Gly Ser
                85                  90                  95

Met Val Ser Lys Gly Glu Glu Leu Phe Thr Gly Val Val Pro Ile Gln
            100                 105                 110

Val Glu Leu Asp Gly Asp Val Asn Gly His Lys Phe Ser Val Ser Gly
        115                 120                 125

Glu Gly Glu Gly Asp Ala Thr Tyr Gly Lys Leu Thr Leu Lys Phe Ile
    130                 135                 140

Cys Thr Thr Gly Lys Leu Pro Val Pro Trp Pro Thr Leu Val Thr Thr
145                 150                 155                 160

Leu Thr Tyr Gly Val Gln Cys Phe Ser Arg Tyr Pro Asp His Met Lys
                165                 170                 175

Gln His Asp Phe Phe Lys Ser Ala Met Pro Glu Gly Tyr Ile Gln Glu
            180                 185                 190

Arg Thr Ile Phe Phe Lys Asp Asp Gly Asn Tyr Lys Thr Arg Ala Glu
        195                 200                 205

Val Lys Phe Glu Gly Asp Thr Leu Val Asn Arg Ile Glu Leu Lys Gly
    210                 215                 220

Ile Asp Phe Lys Glu Asp Gly Asn Ile Leu Gly His Lys Leu Glu Tyr
225                 230                 235                 240

Asn

<210>  4
<211>  243
<212>  PRT
<213>  Artificial Sequence

<220>
<223>  cpBFP

<400>  4

Asn Val Tyr Ile Lys Ala Asp Lys Gln Lys Asn Gly Ile Lys Ala Asn
1               5                   10                  15

Phe Lys Ile Arg His Asn Ile Glu Gly Gly Gly Val Gln Leu Ala Tyr
            20                  25                  30

His Tyr Gln Gln Asn Thr Pro Ile Gly Asp Gly Pro Val Leu Leu Pro
        35                  40                  45

Asp Asn His Tyr Leu Ser Val Gln Ser Ile Leu Ser Lys Asp Pro Asn
    50                  55                  60

Glu Lys Arg Asp His Met Val Leu Leu Glu Phe Val Thr Ala Ala Gly
65                  70                  75                  80

Ile Thr Leu Gly Met Asp Glu Leu Tyr Lys Gly Gly Thr Gly Gly Ser
                85                  90                  95

Glu Ser Met Val Ser Lys Gly Glu Glu Leu Phe Thr Gly Val Val Pro
            100                 105                 110

Ile Gln Val Glu Leu Asp Gly Asp Val Asn Gly His Lys Phe Ser Val
        115                 120                 125

Ser Gly Glu Gly Glu Gly Asp Ala Thr Tyr Gly Lys Leu Thr Leu Lys
    130                 135                 140

Phe Ile Cys Thr Thr Gly Lys Leu Pro Val Pro Trp Pro Thr Leu Val
145                 150                 155                 160

Thr Thr Leu Ser His Gly Val Gln Cys Phe Ser Arg Tyr Pro Asp His
                165                 170                 175

Met Lys Gln His Asp Phe Phe Lys Ser Ala Met Pro Gly Gly Tyr Ile
            180                 185                 190

Gln Glu Arg Thr Ile Phe Phe Lys Asp Asp Gly Asn Tyr Lys Thr Arg
        195                 200                 205

Ala Glu Val Lys Phe Glu Gly Asp Thr Leu Val Asn Arg Ile Glu Leu
    210                 215                 220

Lys Gly Ile Asp Phe Lys Glu Asp Gly Asn Ile Leu Gly His Lys Leu
225                 230                 235                 240

Glu Tyr Asn

<210>  5
<211>  242

<212>  PRT
<213>  Artificial Sequence

<220>
<223>  cpmApple

<400>  5

Val Ser Glu Arg Met Tyr Pro Glu Asp Gly Ala Leu Lys Ser Glu Ile
1               5                   10                  15

Lys Lys Gly Leu Arg Leu Lys Asp Gly Gly His Tyr Ala Ala Glu Val
            20                  25                  30

Lys Thr Thr Tyr Lys Ala Lys Lys Pro Val Gln Leu Pro Gly Ala Tyr
        35                  40                  45

Ile Val Asp Ile Lys Leu Asp Ile Val Ser His Asn Glu Asp Tyr Thr
    50                  55                  60

Ile Val Glu Gln Cys Glu Arg Ala Glu Gly Arg His Ser Thr Gly Gly
65                  70                  75                  80

Met Asp Glu Leu Tyr Lys Gly Gly Thr Gly Gly Ser Leu Val Ser Lys
                85                  90                  95

Gly Glu Glu Asp Asn Met Ala Ile Ile Lys Glu Phe Met Arg Phe Lys
            100                 105                 110

Val His Met Glu Gly Ser Val Asn Gly His Glu Phe Glu Ile Glu Gly
        115                 120                 125

Glu Gly Glu Gly Arg Pro Tyr Glu Ala Phe Gln Thr Ala Lys Leu Lys
    130                 135                 140

Val Thr Lys Gly Gly Pro Leu Pro Phe Ala Trp Asp Ile Leu Ser Pro
145                 150                 155                 160

Gln Phe Met Tyr Gly Ser Lys Ala Tyr Ile Lys His Pro Ala Asp Ile
                165                 170                 175

Pro Asp Tyr Phe Lys Leu Ser Phe Pro Glu Gly Phe Arg Trp Glu Arg
            180                 185                 190

Val Met Asn Phe Glu Asp Gly Gly Ile Ile His Val Asn Gln Asp Ser
        195                 200                 205

Ser Leu Gln Asp Gly Val Phe Ile Tyr Lys Val Lys Leu Arg Gly Thr
    210                 215                 220

Asn Phe Pro Pro Asp Gly Pro Val Met Gln Lys Lys Thr Met Gly Trp
225                 230                 235                 240

Glu Ala

<210>  6
<211>  426
<212>  PRT
<213>  Artificial Sequence

<220>
<223>  optical probe

<400>  6

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Tyr Leu Val Pro Pro Val Phe Ser Gln Leu Thr Glu Gln His Gln
        355                 360                 365

Ala Val Ile Asp Ala Ile Phe Ala Gly Asp Ala Asp Gly Ala Arg Lys
    370                 375                 380

Ala Met Met Ala His Leu Ser Phe Val His Thr Thr Met Lys Arg Phe
385                 390                 395                 400

Asp Glu Asp Gln Ala Arg His Ala Arg Ile Thr Arg Leu Pro Gly Glu
                405                 410                 415

His Asn Glu His Ser Arg Glu Lys Asn Ala
            420                 425

<210>  7
<211>  425
<212>  PRT
<213>  Artificial Sequence

<220>
<223>  optical probe

<400>  7

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Leu Val Pro Pro Val Phe Ser Gln Leu Thr Glu Gln His Gln Ala
        355                 360                 365

Val Ile Asp Ala Ile Phe Ala Gly Asp Ala Asp Gly Ala Arg Lys Ala
    370                 375                 380

Met Met Ala His Leu Ser Phe Val His Thr Thr Met Lys Arg Phe Asp
385                 390                 395                 400

Glu Asp Gln Ala Arg His Ala Arg Ile Thr Arg Leu Pro Gly Glu His
                405                 410                 415

Asn Glu His Ser Arg Glu Lys Asn Ala
            420                 425

<210>  8
<211>  424
<212>  PRT
<213>  Artificial Sequence

<220>
<223>  optical probe

<400>  8

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Val Pro Pro Val Phe Ser Gln Leu Thr Glu Gln His Gln Ala Val
        355                 360                 365

Ile Asp Ala Ile Phe Ala Gly Asp Ala Asp Gly Ala Arg Lys Ala Met
    370                 375                 380

Met Ala His Leu Ser Phe Val His Thr Thr Met Lys Arg Phe Asp Glu
385                 390                 395                 400

Asp Gln Ala Arg His Ala Arg Ile Thr Arg Leu Pro Gly Glu His Asn
                405                 410                 415

Glu His Ser Arg Glu Lys Asn Ala
            420

<210>  9
<211>  423
<212>  PRT
<213>  Artificial Sequence

<220>
<223>  optical probe

<400>  9

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala

20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Pro Pro Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210> 10
<211> 422
<212> PRT
<213> Artificial Sequence

<220>
<223> optical probe

<400> 10

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile

    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Pro Val Phe Ser Gln Leu Thr Glu Gln His Gln Ala Val Ile Asp
        355                 360                 365

Ala Ile Phe Ala Gly Asp Ala Asp Gly Ala Arg Lys Ala Met Met Ala
    370                 375                 380

His Leu Ser Phe Val His Thr Thr Met Lys Arg Phe Asp Glu Asp Gln
385                 390                 395                 400

Ala Arg His Ala Arg Ile Thr Arg Leu Pro Gly Glu His Asn Glu His
                405                 410                 415

Ser Arg Glu Lys Asn Ala
            420

<210>  11
<211>  426
<212>  PRT
<213>  Artificial Sequence

<220>
<223>  optical probe

<400>  11

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu

                85                  90                  95

Gln Ser Ser Val Lys His Ser Arg Gln Arg Met Tyr Tyr Asn Ser Asp
            100                 105                 110

Asn Val Tyr Ile Met Ala Asp Lys Gln Lys Asn Gly Ile Lys Ala Asn
        115                 120                 125

Phe Lys Ile Arg His Asn Val Glu Asp Gly Ser Val Gln Leu Ala Asp
    130                 135                 140

His Tyr Gln Gln Asn Thr Pro Ile Gly Asp Gly Pro Val Leu Leu Pro
145                 150                 155                 160

Asp Asn His Tyr Leu Ser Phe Gln Ser Val Leu Ser Lys Asp Pro Asn
                165                 170                 175

Glu Lys Arg Asp His Met Val Leu Leu Glu Phe Val Thr Ala Ala Gly
            180                 185                 190

Ile Thr Leu Gly Met Asp Glu Leu Tyr Asn Val Asp Gly Gly Ser Gly
        195                 200                 205

Gly Thr Gly Ser Lys Gly Glu Glu Leu Phe Thr Gly Val Val Pro Ile
    210                 215                 220

Leu Val Glu Leu Asp Gly Asp Val Asn Gly His Lys Phe Ser Val Ser
225                 230                 235                 240

Gly Glu Gly Glu Gly Asp Ala Thr Tyr Gly Lys Leu Thr Leu Lys Leu
                245                 250                 255

Ile Cys Thr Thr Gly Lys Leu Pro Val Pro Trp Pro Thr Leu Val Thr
            260                 265                 270

Thr Leu Gly Tyr Gly Leu Lys Cys Phe Ala Arg Tyr Pro Asp His Met
        275                 280                 285

Lys Gln His Asp Phe Phe Lys Ser Ala Met Pro Glu Gly Tyr Val Gln
    290                 295                 300

Glu Arg Thr Ile Phe Phe Lys Asp Asp Gly Asn Tyr Lys Thr Arg Ala
305                 310                 315                 320

Glu Val Lys Phe Glu Gly Asp Thr Leu Val Asn Arg Ile Glu Leu Lys
                325                 330                 335

Gly Ile Asp Phe Lys Glu Asp Gly Asn Ile Leu Gly His Lys Leu Glu
            340                 345                 350

Tyr Asn Leu Val Pro Pro Val Phe Ser Gln Leu Thr Glu Gln His Gln
        355                 360                 365

Ala Val Ile Asp Ala Ile Phe Ala Gly Asp Ala Asp Gly Ala Arg Lys
    370                 375                 380

Ala Met Met Ala His Leu Ser Phe Val His Thr Thr Met Lys Arg Phe
385                 390                 395                 400

Asp Glu Asp Gln Ala Arg His Ala Arg Ile Thr Arg Leu Pro Gly Glu
                405                 410                 415

His Asn Glu His Ser Arg Glu Lys Asn Ala
            420                 425

<210>  12
<211>  425
<212>  PRT
<213>  Artificial Sequence

<220>
<223>  optical probe

<400>  12

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Tyr Asn Ser Asp
            100                 105                 110

Asn Val Tyr Ile Met Ala Asp Lys Gln Lys Asn Gly Ile Lys Ala Asn

115                 120                 125

Phe Lys Ile Arg His Asn Val Glu Asp Gly Ser Val Gln Leu Ala Asp
    130                 135                 140

His Tyr Gln Gln Asn Thr Pro Ile Gly Asp Gly Pro Val Leu Leu Pro
145                 150                 155                 160

Asp Asn His Tyr Leu Ser Phe Gln Ser Val Leu Ser Lys Asp Pro Asn
                165                 170                 175

Glu Lys Arg Asp His Met Val Leu Leu Glu Phe Val Thr Ala Ala Gly
            180                 185                 190

Ile Thr Leu Gly Met Asp Glu Leu Tyr Asn Val Asp Gly Gly Ser Gly
        195                 200                 205

Gly Thr Gly Ser Lys Gly Glu Glu Leu Phe Thr Gly Val Val Pro Ile
    210                 215                 220

Leu Val Glu Leu Asp Gly Asp Val Asn Gly His Lys Phe Ser Val Ser
225                 230                 235                 240

Gly Glu Gly Glu Gly Asp Ala Thr Tyr Gly Lys Leu Thr Leu Lys Leu
                245                 250                 255

Ile Cys Thr Thr Gly Lys Leu Pro Val Pro Trp Pro Thr Leu Val Thr
            260                 265                 270

Thr Leu Gly Tyr Gly Leu Lys Cys Phe Ala Arg Tyr Pro Asp His Met
        275                 280                 285

Lys Gln His Asp Phe Phe Lys Ser Ala Met Pro Glu Gly Tyr Val Gln
    290                 295                 300

Glu Arg Thr Ile Phe Phe Lys Asp Asp Gly Asn Tyr Lys Thr Arg Ala
305                 310                 315                 320

Glu Val Lys Phe Glu Gly Asp Thr Leu Val Asn Arg Ile Glu Leu Lys
                325                 330                 335

Gly Ile Asp Phe Lys Glu Asp Gly Asn Ile Leu Gly His Lys Leu Glu
            340                 345                 350

Tyr Asn Val Pro Pro Val Phe Ser Gln Leu Thr Glu Gln His Gln Ala
        355                 360                 365

Val Ile Asp Ala Ile Phe Ala Gly Asp Ala Asp Gly Ala Arg Lys Ala
    370                 375                 380

Met Met Ala His Leu Ser Phe Val His Thr Thr Met Lys Arg Phe Asp
385                 390                 395                 400

Glu Asp Gln Ala Arg His Ala Arg Ile Thr Arg Leu Pro Gly Glu His
                405                 410                 415

Asn Glu His Ser Arg Glu Lys Asn Ala
            420                 425

<210>  13
<211>  424
<212>  PRT
<213>  Artificial Sequence

<220>
<223>  optical probe

<400>  13

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Tyr Asn Ser Asp
            100                 105                 110

Asn Val Tyr Ile Met Ala Asp Lys Gln Lys Asn Gly Ile Lys Ala Asn
        115                 120                 125

Phe Lys Ile Arg His Asn Val Glu Asp Gly Ser Val Gln Leu Ala Asp
    130                 135                 140

His Tyr Gln Gln Asn Thr Pro Ile Gly Asp Gly Pro Val Leu Leu Pro

145                 150                 155                 160

Asp Asn His Tyr Leu Ser Phe Gln Ser Val Leu Ser Lys Asp Pro Asn
                165                 170                 175

Glu Lys Arg Asp His Met Val Leu Leu Glu Phe Val Thr Ala Ala Gly
            180                 185                 190

Ile Thr Leu Gly Met Asp Glu Leu Tyr Asn Val Asp Gly Gly Ser Gly
        195                 200                 205

Gly Thr Gly Ser Lys Gly Glu Glu Leu Phe Thr Gly Val Val Pro Ile
    210                 215                 220

Leu Val Glu Leu Asp Gly Asp Val Asn Gly His Lys Phe Ser Val Ser
225                 230                 235                 240

Gly Glu Gly Glu Gly Asp Ala Thr Tyr Gly Lys Leu Thr Leu Lys Leu
                245                 250                 255

Ile Cys Thr Thr Gly Lys Leu Pro Val Pro Trp Pro Thr Leu Val Thr
            260                 265                 270

Thr Leu Gly Tyr Gly Leu Lys Cys Phe Ala Arg Tyr Pro Asp His Met
        275                 280                 285

Lys Gln His Asp Phe Phe Lys Ser Ala Met Pro Glu Gly Tyr Val Gln
    290                 295                 300

Glu Arg Thr Ile Phe Phe Lys Asp Asp Gly Asn Tyr Lys Thr Arg Ala
305                 310                 315                 320

Glu Val Lys Phe Glu Gly Asp Thr Leu Val Asn Arg Ile Glu Leu Lys
                325                 330                 335

Gly Ile Asp Phe Lys Glu Asp Gly Asn Ile Leu Gly His Lys Leu Glu
            340                 345                 350

Tyr Asn Pro Pro Val Phe Ser Gln Leu Thr Glu Gln His Gln Ala Val
        355                 360                 365

Ile Asp Ala Ile Phe Ala Gly Asp Ala Asp Gly Ala Arg Lys Ala Met
    370                 375                 380

Met Ala His Leu Ser Phe Val His Thr Thr Met Lys Arg Phe Asp Glu
385                 390                 395                 400

Asp Gln Ala Arg His Ala Arg Ile Thr Arg Leu Pro Gly Glu His Asn
                405                 410                 415

Glu His Ser Arg Glu Lys Asn Ala
            420

<210>  14
<211>  423
<212>  PRT
<213>  Artificial Sequence

<220>
<223>  optical probe

<400>  14

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Tyr Asn Ser Asp
            100                 105                 110

Asn Val Tyr Ile Met Ala Asp Lys Gln Lys Asn Gly Ile Lys Ala Asn
        115                 120                 125

Phe Lys Ile Arg His Asn Val Glu Asp Gly Ser Val Gln Leu Ala Asp
    130                 135                 140

His Tyr Gln Gln Asn Thr Pro Ile Gly Asp Gly Pro Val Leu Leu Pro
145                 150                 155                 160

Asp Asn His Tyr Leu Ser Phe Gln Ser Val Leu Ser Lys Asp Pro Asn
                165                 170                 175

Glu Lys Arg Asp His Met Val Leu Leu Glu Phe Val Thr Ala Ala Gly

180                 185                 190

Ile Thr Leu Gly Met Asp Glu Leu Tyr Asn Val Asp Gly Gly Ser Gly
        195                 200                 205

Gly Thr Gly Ser Lys Gly Glu Glu Leu Phe Thr Gly Val Val Pro Ile
    210                 215                 220

Leu Val Glu Leu Asp Gly Asp Val Asn Gly His Lys Phe Ser Val Ser
225                 230                 235                 240

Gly Glu Gly Glu Gly Asp Ala Thr Tyr Gly Lys Leu Thr Leu Lys Leu
                245                 250                 255

Ile Cys Thr Thr Gly Lys Leu Pro Val Pro Trp Pro Thr Leu Val Thr
            260                 265                 270

Thr Leu Gly Tyr Gly Leu Lys Cys Phe Ala Arg Tyr Pro Asp His Met
        275                 280                 285

Lys Gln His Asp Phe Phe Lys Ser Ala Met Pro Glu Gly Tyr Val Gln
    290                 295                 300

Glu Arg Thr Ile Phe Phe Lys Asp Asp Gly Asn Tyr Lys Thr Arg Ala
305                 310                 315                 320

Glu Val Lys Phe Glu Gly Asp Thr Leu Val Asn Arg Ile Glu Leu Lys
                325                 330                 335

Gly Ile Asp Phe Lys Glu Asp Gly Asn Ile Leu Gly His Lys Leu Glu
            340                 345                 350

Tyr Asn Pro Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  15
<211>  425
<212>  PRT
<213>  Artificial Sequence

<220>
<223>  optical probe

<400>  15

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Leu Tyr Asn Ser
            100                 105                 110

Asp Asn Val Tyr Ile Met Ala Asp Lys Gln Lys Asn Gly Ile Lys Ala
        115                 120                 125

Asn Phe Lys Ile Arg His Asn Val Glu Asp Gly Ser Val Gln Leu Ala
    130                 135                 140

Asp His Tyr Gln Gln Asn Thr Pro Ile Gly Asp Gly Pro Val Leu Leu
145                 150                 155                 160

Pro Asp Asn His Tyr Leu Ser Phe Gln Ser Val Leu Ser Lys Asp Pro
                165                 170                 175

Asn Glu Lys Arg Asp His Met Val Leu Leu Glu Phe Val Thr Ala Ala
            180                 185                 190

Gly Ile Thr Leu Gly Met Asp Glu Leu Tyr Asn Val Asp Gly Gly Ser
        195                 200                 205

Gly Gly Thr Gly Ser Lys Gly Glu Glu Leu Phe Thr Gly Val Val Pro

210                 215                 220

Ile Leu Val Glu Leu Asp Gly Asp Val Asn Gly His Lys Phe Ser Val
225                 230                 235                 240

Ser Gly Glu Gly Glu Gly Asp Ala Thr Tyr Gly Lys Leu Thr Leu Lys
                245                 250                 255

Leu Ile Cys Thr Thr Gly Lys Leu Pro Val Pro Trp Pro Thr Leu Val
            260                 265                 270

Thr Thr Leu Gly Tyr Gly Leu Lys Cys Phe Ala Arg Tyr Pro Asp His
        275                 280                 285

Met Lys Gln His Asp Phe Phe Lys Ser Ala Met Pro Glu Gly Tyr Val
    290                 295                 300

Gln Glu Arg Thr Ile Phe Phe Lys Asp Asp Gly Asn Tyr Lys Thr Arg
305                 310                 315                 320

Ala Glu Val Lys Phe Glu Gly Asp Thr Leu Val Asn Arg Ile Glu Leu
                325                 330                 335

Lys Gly Ile Asp Phe Lys Glu Asp Gly Asn Ile Leu Gly His Lys Leu
            340                 345                 350

Glu Tyr Asn Pro Pro Val Phe Ser Gln Leu Thr Glu Gln His Gln Ala
        355                 360                 365

Val Ile Asp Ala Ile Phe Ala Gly Asp Ala Asp Gly Ala Arg Lys Ala
    370                 375                 380

Met Met Ala His Leu Ser Phe Val His Thr Thr Met Lys Arg Phe Asp
385                 390                 395                 400

Glu Asp Gln Ala Arg His Ala Arg Ile Thr Arg Leu Pro Gly Glu His
                405                 410                 415

Asn Glu His Ser Arg Glu Lys Asn Ala
            420                 425

<210>  16
<211>  425
<212>  PRT
<213>  Artificial Sequence

<220>
<223>  optical probe

<400>  16

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Leu Val Pro Tyr
            100                 105                 110

Asn Ser Asp Asn Val Tyr Ile Met Ala Asp Lys Gln Lys Asn Gly Ile
        115                 120                 125

Lys Ala Asn Phe Lys Ile Arg His Asn Val Glu Asp Gly Ser Val Gln
    130                 135                 140

Leu Ala Asp His Tyr Gln Gln Asn Thr Pro Ile Gly Asp Gly Pro Val
145                 150                 155                 160

Leu Leu Pro Asp Asn His Tyr Leu Ser Phe Gln Ser Val Leu Ser Lys
                165                 170                 175

Asp Pro Asn Glu Lys Arg Asp His Met Val Leu Leu Glu Phe Val Thr
            180                 185                 190

Ala Ala Gly Ile Thr Leu Gly Met Asp Glu Leu Tyr Asn Val Asp Gly
        195                 200                 205

Gly Ser Gly Gly Thr Gly Ser Lys Gly Glu Glu Leu Phe Thr Gly Val
    210                 215                 220

Val Pro Ile Leu Val Glu Leu Asp Gly Asp Val Asn Gly His Lys Phe
225                 230                 235                 240

Ser Val Ser Gly Glu Gly Glu Gly Asp Ala Thr Tyr Gly Lys Leu Thr

245                 250                 255

Leu Lys Leu Ile Cys Thr Thr Gly Lys Leu Pro Val Pro Trp Pro Thr
            260                 265                 270

Leu Val Thr Thr Leu Gly Tyr Gly Leu Lys Cys Phe Ala Arg Tyr Pro
        275                 280                 285

Asp His Met Lys Gln His Asp Phe Phe Lys Ser Ala Met Pro Glu Gly
    290                 295                 300

Tyr Val Gln Glu Arg Thr Ile Phe Phe Lys Asp Asp Gly Asn Tyr Lys
305                 310                 315                 320

Thr Arg Ala Glu Val Lys Phe Glu Gly Asp Thr Leu Val Asn Arg Ile
                325                 330                 335

Glu Leu Lys Gly Ile Asp Phe Lys Glu Asp Gly Asn Ile Leu Gly His
            340                 345                 350

Lys Leu Glu Tyr Asn Val Phe Ser Gln Leu Thr Glu Gln His Gln Ala
        355                 360                 365

Val Ile Asp Ala Ile Phe Ala Gly Asp Ala Asp Gly Ala Arg Lys Ala
    370                 375                 380

Met Met Ala His Leu Ser Phe Val His Thr Thr Met Lys Arg Phe Asp
385                 390                 395                 400

Glu Asp Gln Ala Arg His Ala Arg Ile Thr Arg Leu Pro Gly Glu His
                405                 410                 415

Asn Glu His Ser Arg Glu Lys Asn Ala
            420                 425

<210>  17
<211>  426
<212>  PRT
<213>  Artificial Sequence

<220>
<223>  optical probe

<400>  17

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Leu Val Pro Pro
            100                 105                 110

Tyr Asn Ser Asp Asn Val Tyr Ile Met Ala Asp Lys Gln Lys Asn Gly
        115                 120                 125

Ile Lys Ala Asn Phe Lys Ile Arg His Asn Val Glu Asp Gly Ser Val
    130                 135                 140

Gln Leu Ala Asp His Tyr Gln Gln Asn Thr Pro Ile Gly Asp Gly Pro
145                 150                 155                 160

Val Leu Leu Pro Asp Asn His Tyr Leu Ser Phe Gln Ser Val Leu Ser
                165                 170                 175

Lys Asp Pro Asn Glu Lys Arg Asp His Met Val Leu Leu Glu Phe Val
            180                 185                 190

Thr Ala Ala Gly Ile Thr Leu Gly Met Asp Glu Leu Tyr Asn Val Asp
        195                 200                 205

Gly Gly Ser Gly Gly Thr Gly Ser Lys Gly Glu Glu Leu Phe Thr Gly
    210                 215                 220

Val Val Pro Ile Leu Val Glu Leu Asp Gly Asp Val Asn Gly His Lys
225                 230                 235                 240

Phe Ser Val Ser Gly Glu Gly Glu Gly Asp Ala Thr Tyr Gly Lys Leu
                245                 250                 255

Thr Leu Lys Leu Ile Cys Thr Thr Gly Lys Leu Pro Val Pro Trp Pro
            260                 265                 270

Thr Leu Val Thr Thr Leu Gly Tyr Gly Leu Lys Cys Phe Ala Arg Tyr

275                 280                 285

Pro Asp His Met Lys Gln His Asp Phe Phe Lys Ser Ala Met Pro Glu
    290                 295                 300

Gly Tyr Val Gln Glu Arg Thr Ile Phe Phe Lys Asp Asp Gly Asn Tyr
305                 310                 315                 320

Lys Thr Arg Ala Glu Val Lys Phe Glu Gly Asp Thr Leu Val Asn Arg
                325                 330                 335

Ile Glu Leu Lys Gly Ile Asp Phe Lys Glu Asp Gly Asn Ile Leu Gly
            340                 345                 350

His Lys Leu Glu Tyr Asn Val Phe Ser Gln Leu Thr Glu Gln His Gln
        355                 360                 365

Ala Val Ile Asp Ala Ile Phe Ala Gly Asp Ala Asp Gly Ala Arg Lys
    370                 375                 380

Ala Met Met Ala His Leu Ser Phe Val His Thr Thr Met Lys Arg Phe
385                 390                 395                 400

Asp Glu Asp Gln Ala Arg His Ala Arg Ile Thr Arg Leu Pro Gly Glu
                405                 410                 415

His Asn Glu His Ser Arg Glu Lys Asn Ala
            420                 425

<210>  18
<211>  423
<212>  PRT
<213>  Artificial Sequence

<220>
<223>  optical probe

<400>  18

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu

305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Asn Pro Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  19
<211>  423
<212>  PRT
<213>  Artificial Sequence

<220>
<223>  optical probe

<400>  19

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr

340                 345                 350

Asn Ser Pro Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  20
<211>  423
<212>  PRT
<213>  Artificial Sequence

<220>
<223>  optical probe

<400>  20

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Cys Phe Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met

370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  21
<211>  423
<212>  PRT
<213>  Artificial Sequence

<220>
<223>  optical probe

<400>  21

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Asn Phe Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu

                405                 410                 415

His Ser Arg Glu Lys Asn Ala
            420

<210> 22
<211> 423
<212> PRT
<213> Artificial Sequence

<220>
<223> optical probe

<400> 22

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Asn Tyr Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  23

<211>  423
<212>  PRT
<213>  Artificial Sequence

<220>
<223>  optical probe

<400>  23

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn His Arg Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  24
<211>  423
<212>  PRT
<213>  Artificial Sequence

<220>
<223>  optical probe

<400>  24

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Arg Ile Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210> 25
<211> 423
<212> PRT
<213> Artificial Sequence

<220>
<223> optical probe

<400> 25

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Phe His Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  26
<211>  423
<212>  PRT
<213>  Artificial Sequence

<220>
<223>  optical probe

<400>  26

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Cys Tyr Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210> 27
<211> 423
<212> PRT
<213> Artificial Sequence

<220>
<223> optical probe

<400> 27

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Cys Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  28
<211>  423
<212>  PRT
<213>  Artificial Sequence

<220>
<223>  optical probe

<400>  28

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Met Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  29
<211>  423
<212>  PRT
<213>  Artificial Sequence

<220>
<223>  optical probe

<400>  29

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn His Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
                420

<210>  30
<211>  423
<212>  PRT
<213>  Artificial Sequence

<220>
<223>  optical probe

<400>  30

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Met Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Phe Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  31
<211>  1272

<212>  DNA
<213>  Artificial Sequence

<220>
<223>  nucleic acid sequence of exemplary probe

<400>  31
atggagcaaa acatcgtcca gccgctaaaa acactgatgg ccgatgatcc ggattacagt      60

ttcgatattc tggaagcccg ctacgccatt gaagccagca ccgcatggca tgcggcaatg     120

cgcgccacac ctggcgacaa agaaaagatt cagctttgct ttgaagcaac gctaagtgaa     180

gacccggata tcgcctcaca agcggacgtt cgttttcatc tggcgattgc cgaagcctca     240

cataacatcg tgctgctgca aaccatgcgc ggtttcttcg atgtcctgca atcctcagtg     300

aagcatagcc gtcagcggat gtacaacagc gacaacgtct atatcatggc cgacaagcag     360

aagaacggca tcaaggccaa cttcaagatc cgccacaacg tcgaggacgg cagcgtgcag     420

ctcgccgacc actaccagca gaacaccccc atcggcgacg gccccgtgct gctgcccgac     480

aaccactacc tgagcttcca gtccgtcctg agcaaagacc ccaacgagaa gcgcgatcac     540

atggtcctgc tggagttcgt gaccgccgcc gggatcactc tcggcatgga cgagctgtac     600

aacgtggatg gcggtagcgg tggcaccggc agcaagggcg aggagctgtt caccggggtg     660

gtgcccatcc tggtcgagct ggacggcgac gtaaacggcc acaagttcag cgtgtccggc     720

gagggcgagg gcgatgccac ctacggcaag ctgaccctga agctgatctg caccaccggc     780

aagctgcccg tgccctggcc caccctcgtg accaccctcg gctacggcct gaagtgcttc     840

gcccgctacc ccgaccacat gaagcagcac gacttcttca agtccgccat gcccgaaggc     900

tacgtccagg agcgcaccat cttcttcaag gacgacggca actacaagac ccgcgccgag     960

gtgaagttcg agggcgacac cctggtgaac cgcatcgagc tgaagggcat cgacttcaag    1020

gaggacggca acatcctggg gcacaagctg gagtacaact ttgatgtttt ttcacaactg    1080

accgaacaac atcaggctgt cattgacgcc atttttgccg gtgatgctga cggggcgcgt    1140

aaagcaatga tggcgcacct tagttttgtt cacaccacca tgaaacgatt cgatgaagat    1200

caggctcgcc acgcacggat tacccgcctg cccggtgagc ataatgagca ttcgagggag    1260

aaaaacgcat aa                                                        1272


<210>  32
<211>  28
<212>  DNA
<213>  Artificial Sequence

<220>
<223>  primer


<220>
<221>  misc_feature

<222>  (1)..(2)
<223>  n is a, c, g, or t

<220>
<221>  misc_feature
<222>  (4)..(5)
<223>  n is a, c, g, or t

<400>  32
nnknnkgttt tttcacaact gaccgaac                                          28


<210>  33
<211>  24
<212>  DNA
<213>  Artificial Sequence

<220>
<223>  primer

<400>  33
gttgtactcc agcttgtgcc ccag                                              24


<210>  34
<211>  423
<212>  PRT
<213>  Artificial Sequence

<220>
<223>  optical probe

<400>  34

Met Glu Gln Asn Ile Val Gln Pro Leu Lys Thr Leu Met Ala Asp Asp
1               5                   10                  15


Pro Asp Tyr Ser Phe Asp Ile Leu Glu Ala Arg Tyr Ala Ile Glu Ala
            20                  25                  30


Ser Thr Ala Trp His Ala Ala Met Arg Ala Thr Pro Gly Asp Lys Glu
        35                  40                  45


Lys Ile Gln Leu Cys Phe Glu Ala Thr Leu Ser Glu Asp Pro Asp Ile
    50                  55                  60


Ala Ser Gln Ala Asp Val Arg Phe His Leu Ala Ile Ala Glu Ala Ser
65                  70                  75                  80


His Asn Ile Val Leu Leu Gln Thr Met Arg Gly Phe Phe Asp Val Leu
                85                  90                  95


Gln Ser Ser Val Lys His Ser Arg Gln Arg Phe Tyr Asn Ser Asp Asn
            100                 105                 110


Val Tyr Ile Met Ala Asp Lys Gln Lys Asn Gly Ile Lys Ala Asn Phe

115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn His Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  35
<211>  423
<212>  PRT
<213>  Artificial Sequence

<220>
<223>  optical probe

<400>  35

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Leu Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp

145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn His Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  36
<211>  423
<212>  PRT
<213>  Artificial Sequence

<220>
<223>  optical probe

<400>  36

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Ile Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile

            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn His Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  37
<211>  423
<212>  PRT
<213>  Artificial Sequence

<220>
<223>  optical probe

<400>  37

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Ser Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu

210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn His Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  38
<211>  423
<212>  PRT
<213>  Artificial Sequence

<220>
<223>  optical probe

<400>  38

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Val Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile

245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn His Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  39
<211>  423
<212>  PRT
<213>  Artificial Sequence

<220>
<223>  optical probe

<400>  39

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Leu Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys

275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu
305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Met Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420

<210>  40
<211>  423
<212>  PRT
<213>  Artificial Sequence

<220>
<223>  optical probe

<400>  40

Met Glu Gln Asn Ile Val Gln Pro Leu Lys Thr Leu Met Ala Asp Asp
1               5                   10                  15

Pro Asp Tyr Ser Phe Asp Ile Leu Glu Ala Arg Tyr Ala Ile Glu Ala
            20                  25                  30

Ser Thr Ala Trp His Ala Ala Met Arg Ala Thr Pro Gly Asp Lys Glu
        35                  40                  45

Lys Ile Gln Leu Cys Phe Glu Ala Thr Leu Ser Glu Asp Pro Asp Ile
    50                  55                  60

Ala Ser Gln Ala Asp Val Arg Phe His Leu Ala Ile Ala Glu Ala Ser
65                  70                  75                  80

His Asn Ile Val Leu Leu Gln Thr Met Arg Gly Phe Phe Asp Val Leu
                85                  90                  95

Gln Ser Ser Val Lys His Ser Arg Gln Arg Ala Tyr Asn Ser Asp Asn
            100                 105                 110

Val Tyr Ile Met Ala Asp Lys Gln Lys Asn Gly Ile Lys Ala Asn Phe
        115                 120                 125

Lys Ile Arg His Asn Val Glu Asp Gly Ser Val Gln Leu Ala Asp His
    130                 135                 140

Tyr Gln Gln Asn Thr Pro Ile Gly Asp Gly Pro Val Leu Leu Pro Asp
145                 150                 155                 160

Asn His Tyr Leu Ser Phe Gln Ser Val Leu Ser Lys Asp Pro Asn Glu
                165                 170                 175

Lys Arg Asp His Met Val Leu Leu Glu Phe Val Thr Ala Ala Gly Ile
            180                 185                 190

Thr Leu Gly Met Asp Glu Leu Tyr Asn Val Asp Gly Gly Ser Gly Gly
        195                 200                 205

Thr Gly Ser Lys Gly Glu Glu Leu Phe Thr Gly Val Val Pro Ile Leu
    210                 215                 220

Val Glu Leu Asp Gly Asp Val Asn Gly His Lys Phe Ser Val Ser Gly
225                 230                 235                 240

Glu Gly Glu Gly Asp Ala Thr Tyr Gly Lys Leu Thr Leu Lys Leu Ile
                245                 250                 255

Cys Thr Thr Gly Lys Leu Pro Val Pro Trp Pro Thr Leu Val Thr Thr
            260                 265                 270

Leu Gly Tyr Gly Leu Lys Cys Phe Ala Arg Tyr Pro Asp His Met Lys
        275                 280                 285

Gln His Asp Phe Phe Lys Ser Ala Met Pro Glu Gly Tyr Val Gln Glu
    290                 295                 300

Arg Thr Ile Phe Phe Lys Asp Asp Gly Asn Tyr Lys Thr Arg Ala Glu

305                 310                 315                 320

Val Lys Phe Glu Gly Asp Thr Leu Val Asn Arg Ile Glu Leu Lys Gly
                325                 330                 335

Ile Asp Phe Lys Glu Asp Gly Asn Ile Leu Gly His Lys Leu Glu Tyr
            340                 345                 350

Asn Met Asp Val Phe Ser Gln Leu Thr Glu Gln His Gln Ala Val Ile
        355                 360                 365

Asp Ala Ile Phe Ala Gly Asp Ala Asp Gly Ala Arg Lys Ala Met Met
    370                 375                 380

Ala His Leu Ser Phe Val His Thr Thr Met Lys Arg Phe Asp Glu Asp
385                 390                 395                 400

Gln Ala Arg His Ala Arg Ile Thr Arg Leu Pro Gly Glu His Asn Glu
                405                 410                 415

His Ser Arg Glu Lys Asn Ala
            420